(19) Europäisches Patentamt
European Patent Office
Office européen des brevets

(11) EP 1 908 461 B1

(12) **FASCICULE DE BREVET EUROPEEN**

(45) Date de publication et mention de la délivrance du brevet:
**16.03.2011 Bulletin 2011/11**

(51) Int Cl.:
*A61K 31/135* (2006.01) *A61K 31/165* (2006.01)

(21) Numéro de dépôt: **07123564.2**

(22) Date de dépôt: **16.02.2004**

(54) **Utilisation de l'énantiomère (1S, 2R) du milnacipran pour la préparation d'un médicament**

Verwendung des Enantiomers (1S, 2R) von Milnacipran zur Herstellung eines Arzneimittels

Use of (1S, 2R) enantiomer of milnacipran for the preparation of a medicine

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **14.02.2003 FR 0301849**
**03.06.2003 US 453574**

(43) Date de publication de la demande:
**09.04.2008 Bulletin 2008/15**

(60) Demande divisionnaire:
**10182592.5**

(62) Numéro(s) de document de la (des) demande(s) initiale(s) en application de l'article 76 CBE:
**04711387.3 / 1 601 349**

(73) Titulaire: **PIERRE FABRE MEDICAMENT**
**92100 Boulogne-Billancourt (FR)**

(72) Inventeurs:
- **Deregnaucourt, Jean**
**75014, PARIS (FR)**
- **Grosse, Richard**
**45520, GIDY (FR)**

(74) Mandataire: **Ahner, Francis et al**
**Cabinet Régimbeau**
**20, rue de Chazelles**
**75847 Paris Cedex 17 (FR)**

(56) Documents cités:
**FR-A- 2 759 906**

- **BONNAUD B ET AL: "1-Aryl-2-(aminomethyl) cyclopropanecarboxy lic acid derivatives. A new series of potential antidepressants" JOURNAL OF MEDICINAL CHEMISTRY, AMERICAN CHEMICAL SOCIETY. WASHINGTON, US, vol. 30, no. 2, 1987, pages 318-325, XP002282456 ISSN: 0022-2623**
- **DEPREZ D ET AL: "Which bioequivalence study for a racemic drug? Application to milnacipran." EUROPEAN JOURNAL OF DRUG METABOLISM AND PHARMACOKINETICS, vol. 23, no. 2, 1998, pages 166-171, XP008021090 ISSN: 0378-7966**
- **SPENCER CAROLINE M ET AL: "Milnacipran: A review of its use in depression." DRUGS, vol. 56, no. 3, 1998, pages 405-427, XP008021105 ISSN: 0012-6667**
- **PUOZZO C ET AL: "Pharmacokinetics of milnacipran in renal impairment." EUROPEAN JOURNAL OF DRUG METABOLISM AND PHARMACOKINETICS. SWITZERLAND 1998 APR-JUN, vol. 23, no. 2, avril 1998 (1998-04), pages 280-286, XP008021091 ISSN: 0398-7639**
- **VIAZZO P ET AL: "Microbiological Transformations 34: Enantioselective Hydrolysis of a Key-Lactone Involved in the Synthesis of the Antidepressant Milnacipran ?" TETRAHEDRON LETTERS, ELSEVIER SCIENCE PUBLISHERS, AMSTERDAM, NL, vol. 37, no. 26, 24 juin 1996 (1996-06-24), pages 4519-4522, XP004029055 ISSN: 0040-4039**
- **SHUTO SATOSHI ET AL: "-1-phenyl-2-((S)-1-aminopropyl)-N,N-dieth ylcyclopropanecarboxamide (PPDC), a new class of NMDA-receptor antagonist: Molecular design by a novel conformational restriction strategy." JAPANESE JOURNAL OF PHARMACOLOGY, vol. 85, no. 3, mars 2001 (2001-03), pages 207-213, XP008021088 ISSN: 0021-5198**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne l'énantiomère (1S,2R) du Milnacipran (Z(±)-2-(amino méthyl)-N,N-diéthyl- 1-phénylcyclopropanecarboxamide), ou un de ses sels pharmaceutiquement acceptables, pour son utilisation comme médicament destiné à prévenir ou traiter la dépression, les états dépressifs, la fibromyalgie, le syndrome de fatigue chronique, la douleur, chez des patients présentant des antécédents cardiovasculaires et/ou atteints de troubles cardiovasculaires, administré à une dose comprise entre 0.01 mg et 10 mg/kg de poids corporel par jour en une ou plusieurs prises. Plus particulièrement, l'énantiomère selon l'invention est destiné à traiter la dépression, les syndromes de fatigue chronique et l'incontinence urinaire.

Le Milnacipran (Z (±)-2-(amino méthyl)-N,N-diéthyl-1-phényl cyclopropane carboxamide), molécule synthétisée au Centre de Recherche PIERRE FABLE MEDICAMENT (Castres, France), également appelé TN-912, Dalcipran, Minalcipran, Midalcipran ou Midalipran est connu comme inhibiteur double de la recapture de la sérotonine (5-HT) et de la noradrénaline (NA). Le Milnacipran et son procédé de préparation sont décrits dans le brevet US n° 4,478,836. D'autres informations relatives au Milnacipran peuvent être trouvées dans la douzième édition de l'index Merck, sous l'entrée n° 6 281.

Les inhibiteurs doubles de la recapture de la sérotonine et de la noradrénaline correspondent à une classe bien connue d'agents antidépresseurs qui inhibent de manière sélective la recapture à la fois de la sérotonine et de la noradrénaline. A titre d'exemple, la venlafaxine et la duloxétine sont également des inhibiteurs doubles de la sérotonine et de la noradrénaline. Des études ont montré que le ratio entre l'inhibition de la recapture de la noradrénaline et l'inhibition de la sérotonine par le Milnacipran est d'environ 2 :1 (Moret et al., 1985 Neuropharmacology 24(12) : 1211-1219 ; Palmier et al., 1989, Eur J Clin Pharmacol 37 : 235-238).

Le brevet US 4,478,836 décrit l'utilisation du Milnacipran pour le traitement de pathologies du système nerveux central, notamment de la dépression. La demande de brevet WO01/26623 décrit l'utilisation du Milnacipran en association avec de la phénylalanine et de la tyrosine dans des indications telles le traitement de la fatigue, des syndromes associés à la douleur, du syndrome de fatigue chronique, de la fibromyalgie, du syndrome de l'intestin irritable. La demande de brevet WO01/62236 décrit une composition comprenant du Milnacipran en association avec un ou plusieurs agents anti-muscariniques dans un grand nombre d'indications dont la dépression. La demande WO97/35574 décrit une composition pharmaceutique contenant du Milnacipran et de l'idazoxan comme produit de combinaison pour une utilisation simultanée, séparée ou étalée dans le temps pour traiter la dépression et ses différentes formes, ainsi que les pathologies dans lesquelles les antidépresseurs sont utilisés. Le Milnacipran est également utilisé dans une indication de traitement de l'incontinence urinaire (FR 2 759 290).

La molécule de Milnacipran possède deux carbones asymétriques conduisant à deux configurations spatiales différentes (1S,2R) et (1R,2S). Ces configurations spatiales étant non superposables, la molécule de Milnacipran présente donc une isomérie optique.

Le chlorhydrate de Milnacipran existe ainsi sous la forme de deux énantiomères optiquement actifs : l'énantiomère dextrogyre ou encore chlorhydrate de Z-(1S,2R)-2-(amino méthyl)-N,N-diéthyl-1-phényl cyclopropane carboxamide et l'énantiomère lévogyre chlorhydrate de Z-(1R, 2S)-2-(amino méthyl)-N, N-diethyl-1 -phényl cyclopropane carboxamide. Le Milnacipran sous sa forme chlorhydrate (encore appelée F2207) est actuellement commercialisé (IXEL, PIERRE FABRE MEDICAMENT, France) sous forme de mélange racémique en tant que médicament antidépresseur sérotoninergique-noradrénergique. F2695 et F2696 désignent respectivement les énantiomères (1S,2R) (dextrogyre) et (1R,2S) (lévogyre) du chlorhydrate de Milnacipran (F2207) :

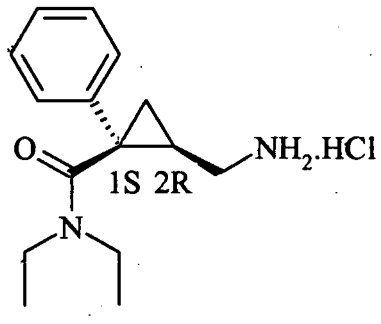

F2695

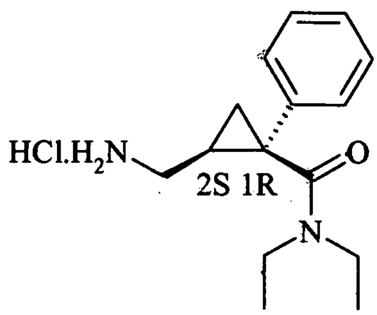

F2696

Ces deux énantiomères peuvent être séparés et isolés selon des procédés décrits dans la littérature (Bonnaud et al., 1985, Journal of Chromatography, Vol. 318 : 398-403 ; Shuto et al., Tetrahedron letters, 1996 Vol. 37 :641-644 ; Grard et al., 2000, Electrophoresis 2000 21 : 3028-3034; Doyle et Hu, 2001, Advanced Synthesis and Catalysis, Vol. 343 : 299-302).

Les inventeurs ont maintenant réalisé une étude pharmacocinétique chez l'homme du racémate et des deux énantiomères du Milnacipran qui met en oeuvre des méthodes de dosage d'énantio-sélectivité. Ils ont ainsi démontré l'absence de racémisation des énantiomères *in vivo.*

Par ailleurs, bien que le racémate ait été résolu, aucune analyse des propriétés pharmacologiques et toxicologiques des deux énantiomères n'a été réalisée, qui utilise les méthodes modernes actuellement à disposition telles les mesures cardiovasculaires par télémétrie, ou les analyses de prédictivité pharmaco-toxico-génomique *in vitro.*

Les antidépresseurs, comme tout principe actif, peuvent générer des effets indésirables ou certaines toxicités qui découlent pour l'essentiel des propriétés pharmacologiques de ces médicaments, mais également de la posologie, de la variabilité individuelle du patient (polymorphisme génétique, insuffisance organique, sexe, âge) ou d'interactions médicamenteuses. Ainsi les antidépresseurs représentent la troisième classe de produits responsables d'intoxication, après les hypnotiques et les tranquillisants (Nores et al., 1987 Thérapie 42 : 555-558). Le risque de surdosage avec des antidépresseurs est grave puisqu'il peut conduire à la mort. Parmi les causes d'intoxications aiguës par les antidépresseurs, il convient de citer l'ingestion involontaire par les enfants (d'autant que certains antidépresseurs sont utilisés pour le traitement de l'énurésie), la tentative de suicide, le surdosage involontaire par le médecin, la co-médication associée chez la personne âgée, les modifications physiologiques et pharmacocinétiques liés à l'âge (insuffisance cardiaque, insuffisance hépatique et/ou rénale ...), un métabolisme ralenti d'origine génétique ou médicamenteuse (inhibition enzymatique). Après les enfants, la personne âgée constitue donc la seconde population à risque parmi les patients traités. Ces personnes ont des concentrations plasmatiques plus élevées, liées à une clairance rénale et/ou hépatique amoindrie, et les risques d'intoxication y sont plus graves (Meadoer-Woodruff et al., 1988 J. Clim. Psychopharmacol. 8 : 28-32).

Les effets secondaires indésirables, généralement bénins, observés durant le traitement par le Milnacipran sont surtout notés durant la première voire les deux premières semaines du traitement et s'estompent par la suite, parallèlement à l'amélioration de l'épisode dépressif. Les évènements indésirables les plus communément rapportés en mono-thérapie ou lors d'association avec d'autres psychotropes sont des vertiges, une hyper-sudation, l'anxiété, des bouffées de chaleur, et de la dysurie. Certains effets indésirables moins communément rapportés sont des nausées, des vomissements, une sécheresse buccale, une constipation, des tremblements, des palpitations, une agitation, des éruptions cutanées. Il est par ailleurs connu que chez les patients présentant des antécédents cardiovasculaires ou recevant simultanément un traitement à visée cardiaque, l'incidence des effets indésirables de nature cardiovasculaire (hypertension, hypotension, hypotension orthostatique, palpitations) peut être augmentée par le Milnacipran. Chez les patients hypertendus ou atteints de cardiopathies, il est ainsi recommandé de renforcer la surveillance clinique car le Milnacipran sous forme de mélange racémique est susceptible d'augmenter la fréquence cardiaque. Ainsi, lors de rares cas de surdosage observés avec le Milnacipran (aux doses de 800 mg à 1 g) en mono-thérapie, les principaux symptômes observés sont les vomissements, les troubles respiratoires et une tachycardie (Dictionnaire Vidal, 78$^{ème}$ édition, 2002). Un autre effet indésirable exceptionnellement induit par le Milnacipran est une élévation élevée des transaminases pouvant traduire une certaine toxicité hépatique.

De fait, les populations à risque susceptibles de développer un certain nombre de manifestations cliniques indésirables au cours ou à la suite d'un traitement par le Milnacipran, sont les enfants, les personnes âgées, les insuffisants hépatiques et/ou rénaux, les patients recevant un traitement induisant des toxicités organiques et/ou tissulaires, notamment des toxicités hépatiques et/ou rénales, les patients recevant un traitement à visée cardiaque ou induisant des effets secondaires cardiovasculaires, les patients présentant des antécédents cardiovasculaires et/ou atteints de troubles cardiovasculaires, notamment les patients présentant des troubles du rythme cardiaque, de la pression artérielle (patients hypo- ou hypertendus) ou les patients atteints de cardiopathies.

Dans un souci de prévenir, toujours plus avant, la survenue d'éventuels effets secondaires susceptibles de constituer un risque, aussi minime soit-il, pour la santé des patients traités au Milnacipran, les inventeurs ont maintenant découvert de manière surprenante et inattendue que l'énantiomère (1S,2R) du Milnacipran, qui présente l'essentiel de l'activité d'inhibition sélective de la re-capture de la sérotonine et de la noradrénaline, induit moins d'effets secondaires de nature cardiovasculaire et de toxicité organique et/ou tissulaire, notamment hépatique, que le mélange racémique. En particulier, les inventeurs ont découvert que l'administration chez le chien de l'énantiomère (1S,2R) du Milnacipran provoque une augmentation moindre de la fréquence cardiaque et de la pression artérielle, en particulier de la pression artérielle diastolique, que celle susceptible d'être provoquée par l'administration du mélange racémique. Les inventeurs ont en outre découvert que l'énantiomère (1S,2R) du chlorhydrate de Milnacipran (F2695) présente un meilleur profil toxico-génomique que l'énantiomère (1R,2S) du chlorhydrate de Milnacipran (F2696) sur un modèle expérimental d'hépatocytes primaires de rat. Les inventeurs ont en outre démontré que l'énantiomère (1R,2S) (F2696) présente un profil toxico-génomique semblable à celui obtenu avec la Clomipramine, utilisée comme psychotrope de référence connue pour sa relative hépato-toxicité.

La présente invention a donc pour objet l'énantiomère (1S,2R) du Milnacipran (Z($\pm$)-2-(amino méthyl)-N,N-diéthyl- 1-phénylcyclopropanecarboxamide), ou un de ses sels pharmaceutiquement acceptables, pour son utilisation comme médicament destiné à prévenir ou traiter la dépression, les états dépressifs, la fibromyalgie, le syndrome de fatigue chronique, la douleur, chez des patients présentant des antécédents cardiovasculaires et/ou atteints de troubles cardiovasculaires, administré à une dose comprise entre 0.01 mg et 10 mg/kg de poids corporel par jour en une ou plusieurs prises.

On entend par «troubles cardiovasculaires», des effets secondaires cardiovasculaires indésirables du médicament administré en mono-thérapie ou en association avec d'autres principes actifs.

Au sens de la présente invention, on entend par «effet secondaire», l'activité prévisible d'un médicament dans un domaine autre que celui pour lequel il est administré, qui peut être gênante ou indésirable lorsqu'elle limite l'utilisation du médicament.

On entend par «toxicité», la propriété d'un médicament d'entraîner des effets nocifs au niveau organique et/ou tissulaire, notamment au niveau des organes ou tissus impliqués dans le métabolisme du Milnacipran, notamment le métabolisme hépatique et/ou rénal du Milnacipran, et plus particulièrement lors du premier passage du Milnacipran au niveau hépatique. De préférence, la toxicité organique est la toxicité cardiaque et la dite toxicité tissulaire est la toxicité hépatique et/ou rénale.

Dans le cadre de la présente invention, on entend par « tout en limitant les risques de troubles cardiovasculaires» ou « tout en limitant les risques de toxicité » le fait d'empêcher que ces risques n'augmentent significativement chez un patient suite à l'administration du médicament.

Dans le cadre de la présente invention, «énantiomère (1S,2R) du Milnacipran» désigne l'énantiomère (1S,2R) du Milnacipran, ainsi que les sels pharmaceutiquement acceptables de celui-ci. De préférence, il s'agit de l'énantiomère (1S,2R) du chlorhydrate de Milnacipran (F2695). «Enantiomère (1R,2S) du Milnacipran» désigne l'énantiomère (1R,2S) du Milnacipran, ainsi que les sels pharmaceutiquement acceptables de celui-ci tels que le chlorhydrate (F2696). «Mélange racémique» désigne un mélange 50:50 en poids d'énantiomère (1S,2R) du Milnacipran et d'énantiomère (1R,2S) du Milnacipran, ainsi que les sels pharmaceutiquement acceptables de ceux-ci.

Il existe des métabolites, de préférence les métabolites actifs *in vivo* du Milnacipran, et leurs sels pharmaceutiquement acceptables, tels que :

o le chlorhydrate de l'acide Z-($\pm$)phényl-1 aminométhyl-2 cyclopropane carboxylique (F1567) :

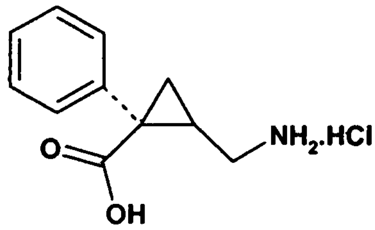

F1567

| | |
|---|---|
| Masse moléculaire : | 277,7 |
| Caractéristiques : | cristaux blancs |
| Point de fusion: | 230 °C |
| Chromatographie sur plaque : | support : silice |
| | Solvant : Butanol/Ethanol/eau (6/2/2) |
| | Révélation : Ultra-violet et ninhydrine |
| | Rf : 0,6 |

o le (±)phényl-3 méthylène-3-4 pyrrolidone-3 (F1612) :

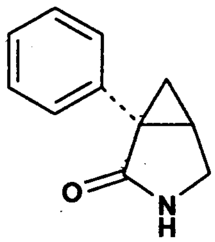

F1612

| | |
|---|---|
| Masse moléculaire : | 173,2 |
| Caractéristiques : | cristaux blancs |
| Point de fusion : | 70 °C |
| Chromatographie sur plaque : | support : silice |
| | Solvant : Benzène/dioxane/éthanol (90/25/4) |
| | Révélation : Ultra-violet et iode |
| | Rf : 0,46 |

o le chlorhydrate de Z(±)-(para-hydroxyphényl)-1 diéthylaminocarbonyl-1 aminométhyl-2 cyclopropane (F2782) :

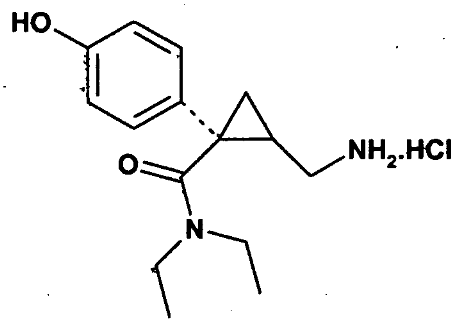

F2782

| | |
|---|---|
| Masse moléculaire : | 298,82 |
| Caractéristiques : | cristaux blancs |
| Point de fusion : | 250 °C |
| Chromatographie sur plaque : | support : silice |
| | Solvant : Butanol/Ethanol/eau (6/2/2) |
| | Révélation : Ultra-violet et iode - ninhydrine |
| | Rf : 0,42 |

o l'oxalate acide de Z(±)-phényl-1-éthylamino carbonyl-1 aminométhyl-2 cyclopropane (F2800) :

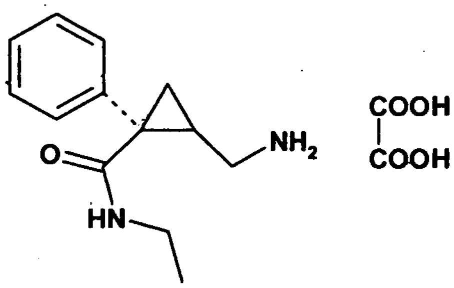

F2800

| | |
|---|---|
| Masse moléculaire : | 308,33 |
| Caractéristiques : | cristaux blancs |
| Point de fusion: | 150 °C |
| Chromatographie sur plaque : | support : silice |
| | Solvant : $CHCl_3$/méthanol/$NH_4OH$ (90/9/1) |
| | Révélation : Ultra-violet et ninhydrine |
| | Rf : 0,40 |

o le chlorhydrate de Z(±)-phényl-1 aminocarbonyl-1 aminométhyl-2 cyclopropane (F2941)

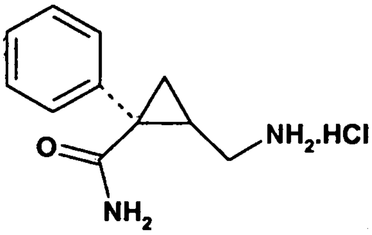

F2941

| | |
|---|---|
| Masse moléculaire : | 226,74 |
| Caractéristiques : | cristaux blancs |
| Point de fusion : | 245 °C |
| Chromatographie sur plaque : | support : silice |
| | Solvant : $CHCl_3$/méthanol/$NH_4OH$ (80/18/2) |
| | Révélation : Ultra-violet et ninhydrine |
| | Rf : 0,30 |

Tout comme le Milnacipran, ces métabolites présentent deux carbones asymétriques conduisant à deux configurations spatiales différentes (1S,2R) et (1R,2S) Ces configurations spatiales étant non superposables, ces métabolites présentent aussi une isomérie optique. Le ratio des deux énantiomères du métabolite du Milnacipran dans le mélange d'énantiomères est tel que décrit précédemment pour les énantiomères du Milnacipran.

Par métabolite actif, on entend désigner un dérivé provenant de la métabolisation du Milnacipran in vitro ou in vivo et qui présente une aptitude à inhiber la recapture de la sérotonine et de la noradrénaline ; de préférence, il s'agit du F2782, F2941, F2800, F1612 et F1567

«Sel pharmaceutiquement acceptable» désigne tous les sels qui conservent l'efficacité et les propriétés d'un principe actif et qui ne présentent pas d'effets secondaires. De préférence, il s'agit de sels d'acides minéraux ou organiques pharmaceutiquement acceptables. A titre d'exemples préférés, mais non limitatifs, on mentionnera les halogénohydrates, tels que chlorhydrate et le bromhydrate, le fumarate, le maléate, l'oxalate, le citrate, le méthane sulfonate, le glutamate, le tartrate, le mésylate, et leurs hydrates éventuels.

L'énantiomère selon l'invention, de préférence l'énantiomère F2695 substantiellement pur, est administré à tout type de patients nécessitant un tel traitement, que ce soit dans un but thérapeutique et/ou prophylactique. Dans un but thérapeutique, on vise l'éradication ou l'amélioration de l'affection à traiter et/ou d'un ou plusieurs symptôme(s) associé(s). Dans un but prophylactique, on vise la prévention de l'apparition de l'affection à traiter et/ou d'un ou plusieurs symptôme(s) associé(s). Néanmoins, l'énantiomère selon l'invention est adapté à des populations de patients à risque qui seraient susceptibles de développer certaines manifestations cliniques indésirables au cours ou à la suite d'un traitement par le Milnacipran sous forme racémique. Il s'agit des patients présentant des antécédents cardiovasculaires (par exemple l'infarctus du myocarde) et/ou atteints de troubles cardiovasculaires, tels des patients présentant des troubles du rythme cardiaque (tachycardie, bradycardie, palpitations).

Parmi les multiples pathologies ou affections qui présentent comme symptôme des troubles du rythme cardiaque et pour lesquelles la présente invention est particulièrement adaptée au traitement des patients à risque qui en sont affectés, il convient de citer plus particulièrement la tachycardie qui correspond à une accélération du rythme des battements cardiaques (la tachycardie est modérée lorsque les pulsations sont de 80 à 100 par minutes, intense quand elles dépassent 100), les palpitations, les extrasystoles (sporadiques, fréquentes ou au cours de l'infarctus du myocarde), la fibrillation auriculaire, le flutter et la tachysystolie auriculaires, la bradycardie, l'insuffisance cardiaque, et l'infarctus du myocarde.

Parmi les multiples pathologies qui présentent comme symptôme des troubles de la pression artérielle et pour lesquelles la présente invention est particulièrement adaptée au traitement des patients à risque qui en sont affectés, il convient de citer plus particulièrement : l'hypertension artérielle, l'hypertension artérielle maligne, l'hypertension artérielle pulmonaire, l'hypertension portale, l'hypertension paroxystique essentielle, l'hypotension, l'hypotension orthostatique, l'hypertension intra-crânienne.

Avantageusement, les troubles cardiovasculaires dont les risques peuvent être limités par l'administration du mélange d'énantiomères selon l'invention, et de préférence par l'administration de l'énantiomère F2695 substantiellement pur, sont :

➢ l'élévation de la pression artérielle diastolique et/ou systolique mesurée en millimètres de mercure (mm de Hg) ; plus particulièrement, il s'agit de l'augmentation de la pression cardiaque diastolique, et/ou,

➢ des troubles du rythme cardiaque, notamment une augmentation de la fréquence cardiaque chez le patient.

La pression artérielle systolique est la valeur maximale de la pression artérielle, et elle correspond au moment où on entend les premiers battements cardiaques au niveau de l'artère humérale lors de la mesure de la pression artérielle. La systole est la période de la révolution cardiaque au cours de laquelle les cavités du coeur se contractent, entraînant ainsi l'éjection du sang. La pression artérielle diastolique est la valeur minimale de la pression artérielle, qui correspond à la disparition des bruits cardiaques au niveau de l'artère humérale lorsque l'on dégonfle le brassard tensionnel lors de la mesure de la pression artérielle. La diastole est la période de la révolution cardiaque au cours de laquelle les cavités du coeur se remplissent de sang. L'élévation de la pression systolique et/ou diastolique implique l'élévation de la tension artérielle qui caractérise l'hypertension artérielle systémique (et ses variantes) dont les symptômes peuvent être les suivants : maux de tête, fatigue, troubles sensoriels légers tels des vertiges, des bourdonnements d'oreilles, des palpitations, un saignement de nez, de la confusion ou de la somnolence, des crampes, engourdissements ou des fourmillements dans les pieds et les mains. L'hypertension artérielle systémique (et ses variantes) peut aboutir à des complications graves, voire parfois mortelles : accidents neurologiques d'origine vasculaire, insuffisance ventriculaire gauche, insuffisance rénale, cardiopathies ischémiques (infarctus du myocarde, angor et leurs variantes). Selon les recommandations actuelles, on considère qu'un patient est affecté d'hypertension artérielle lorsque la pression artérielle est supérieure à 90 mm d'Hg pour la pression diastolique et 140 mm d'Hg pour la pression systolique.

La toxicité dont les risques peuvent être limités par l'administration de l'énantiomère selon l'invention est avantageusement la toxicité organique, notamment la toxicité cardiaque, et/ou la toxicité tissulaire, notamment la toxicité hépatique et/ou rénale. Cette toxicité tissulaire peut être révélée par la présence d'ictères ou par des marqueurs biologiques.

Il entre également dans la portée de la présente invention d'utiliser de l'énantiomère selon l'invention en médecine vétérinaire pour le traitement d'animaux, notamment d'animaux domestiques ou d'élevage nécessitant un tel traitement.

De par leurs propriétés pharmacologiques, notamment d'inhibiteurs doubles de la re-capture de la sérotonine (5-HT) et de la noradrénaline (NA), l'énantiomère selon l'invention est particulièrement utile à la préparation de médicaments destinés au traitement préventif et/ou curatif de nombreuses pathologies ou affections (syndrome) décrites ci-après tout en limitant les risques de troubles cardiovasculaires et/ou tout en limitant la toxicité organique et/ou tissulaire, notamment la toxicité cardiaque hépatique et/ou rénale.

Parmi ces pathologies ou affections, il convient de citer les pathologies du système nerveux central telles que définies dans « The Diagnostic and Statistical Manual of Mental Disorders -IV (DSM-IV), 1995 American Psychiatric Association ». A titre d'exemples illustratifs et non limitatifs, il convient de citer, la dépression, notamment la dépression profonde, la dépression résistante, la dépression du sujet âgé, la dépression psychotique, la dépression induite par des traitements interféron, l'état dépressif, le syndrome maniaco-dépressif, les épisodes dépressifs saisonniers, les épisodes dépressifs liés à une condition médicale générale, les épisodes dépressifs liés à des substances agissant sur l'humeur, le syndrome bipolaire, la schizophrénie, l'anxiété généralisée, les états de morosité et de marasme, les maladies liées au stress, les attaques de panique, la phobie, notamment l'agoraphobie, les troubles obsessionnels compulsifs, les troubles de la conduite, les troubles oppositionnels, les syndromes de stress post-traumatiques, la dépression du système immunitaire, la fatigue et les syndromes de douleur qui l'accompagnent, le syndrome de fatigue chronique, la fibromyalgie, et autres pathologies de nature somatique fonctionnelle, l'autisme, les pathologies caractérisées par une absence d'attention dues à des conditions médicales générales, les troubles de l'attention dus à de l'hyperactivité, les troubles des conduites alimentaires, la névrose boulimique, la névrose anorexique, l'obésité, les désordres psychotiques, l'apathie, la migraine, la douleur, et notamment la douleur chronique, le syndrome du colon irritable, les maladies cardiovasculaires, et notamment le syndrome anxio-dépressif dans l'infarctus du myocarde ou dans l'hypertension, les maladies neuro-dégénératives et les syndromes anxio-dépressifs associés (maladie d'Alzheimer, Chorée de Huntington, maladie de Parkinson), les incontinences urinaires, notamment l'incontinence urinaire liée au stress et l'énurésie, la dépendance aux drogues, et notamment l'anxio-dépendance au tabac, notamment à la nicotine, à l'alcool, aux stupéfiants, aux drogues, aux antalgiques lors du sevrage de ces états de dépendance.

Plus particulièrement, la présente invention a pour objet l'utilisation de l'énantiomére selon l'invention, de préférence l'énantiomère F2695 substantiellement pur, pour la préparation d'un médicament destiné à prévenir ou traiter la dépression ou l'état dépressif tout en limitant les risques de troubles cardiovasculaires et/ou tout en limitant la toxicité organique et/ou tissulaire, notamment la toxicité hépatique et/ou rénale. Dans le cadre de la présente invention, on entend par « dépression » un ensemble de symptômes comprenant d'une part un aspect psychique constitué de troubles de l'humeur avec pessimisme, douleur morale, idées de mort et de suicide, inhibition psychique, et d'autre part un aspect physique d'inhibition motrice constitué notamment d'un ralentissement moteur, de troubles de l'appétit, de constipation, de troubles du sommeil et de la régulation du poids. La dépression correspond donc à un état psychique pathologique

associant une modification pénible de l'humeur et un ralentissement de l'activité intellectuelle et motrice. On entend par « état dépressif » un état mental caractérisé par un fléchissement du tonus neuropsychique, se manifestant par la lassitude, la fatigabilité, le découragement et la tendance au pessimisme et s'accompagnant quelquefois d'anxiété.

Egalement, la présente invention a plus particulièrement pour objet l'utilisation de l'énantiomère selon l'invention, de préférence l'énantiomère F2695 substantiellement pur, pour la préparation d'un médicament destiné à prévenir ou traiter la fibromyalgie et/ou le syndrome de fatigue chronique tout en limitant les risques de troubles cardiovasculaires et/ou tout en limitant la toxicité organique et/ou tissulaire, notamment la toxicité hépatique et/ou rénale. Le syndrome de fibromyalgie est un syndrome chronique caractérisé par une sensation de douleur ou de brûlure avec enraidissement matinal touchant principalement les tissus fibreux articulaires et périarticulaires, et par un sentiment de fatigue profonde. La fibromyalgie comporte un ensemble de symptômes. Les plus fréquents sont un sommeil non réparateur, des maux de tête, des troubles digestifs, un état dépressif, des spasmes musculaires, des douleurs au visage, des engourdissements, etc. Le syndrome de fatigue chronique est caractérisé par un état d'épuisement ou de fatigue. Les symptômes les plus courants sont un état de faiblesse, des spasmes et/ou des douleurs musculaires, un besoin de sommeil excessif, de la fièvre, une angine, des pertes de mémoire et/ou des problèmes de concentration, des insomnies, une dépression.

Egalement, la présente invention a plus particulièrement pour objet l'utilisation de l'énantiomère selon l'invention, de préférence l'énantiomère F2695 substantiellement pur, pour la préparation d'un médicament destiné à prévenir ou traiter douleur et notamment la douleur chronique tout en limitant les risques de troubles cardiovasculaires et/ou tout en limitant la toxicité organique et/ou tissulaire, notamment la toxicité hépatique et/ou rénale. La douleur peut être associée à différentes pathologies et/ou blessures. Elle peut être aiguë ou chronique. Des études épidémiologiques ont démontré les relations existant entre des états de douleurs chroniques et les anxio-dépressions. Ainsi, des patients souffrant de douleur chronique peuvent développer des problèmes émotionnels qui conduisent à une dépression, et, dans le pire des cas, à une tentative de suicide. On considère qu'un patient est atteint de douleurs chroniques s'il se plaint de souffrir depuis une période excédant six mois. Parmi les douleurs chroniques il convient de citer à titre d'exemple illustratif et non limitatif, les douleurs associées à la fibromyalgie et/ou provenant de tissus fibreux, des muscles, des tendons, des ligaments et autres sites, les douleurs abdominales et les diarrhées dans le syndrome du colon irritable, et également les douleurs du bas du dos.

Egalement, la présente invention a plus particulièrement pour objet l'utilisation de l'énantiomère selon l'invention, de préférence l'énantiomère F2695 substantiellement pur, pour la préparation d'un médicament destiné à prévenir ou traiter les incontinences urinaires, notamment l'incontinence urinaire liée au stress et l'énurésie, tout en limitant les risques de troubles cardiovasculaires et/ou tout en limitant la toxicité organique et/ou tissulaire, notamment la toxicité hépatique et/ou rénale.

Le traitement prophylactique et thérapeutique des pathologies ci-dessus est réalisé en délivrant à un animal, de préférence l'homme, une quantité thérapeutiquement efficace de l'énantiomère selon l'invention, de préférence l'énantiomère F2695 substantiellement pur, seul ou en association avec au moins un autre principe actif. Dans la plupart des cas, il s'agit de l'homme, mais le traitement est également adapté aux animaux, notamment aux animaux d'élevage (bétail, rongeurs, volailles, poissons,...) et aux animaux de compagnie (chiens, chats, lapins, chevaux,...).

L'énantiomère (1S,2R) du Milnacipran ainsi que ses sels pharmaceutiquement acceptables, tel que précédemment décrit, est avantageusement administré à des patients recevant simultanément, séparément ou de manière décalée dans le temps au moins un second composé actif dans le traitement des pathologies précédemment citées.

De manière préférée, la présente invention a également pour objet l'utilisation comme médicament:

a) ledit énantiomère (1S,2R) du Milnacipran ainsi que ses sels pharmaceutiquement acceptables, et
b) d'au moins un composé actif choisi parmi les psychotropes, notamment les anti-dépresseurs, et les agent anti-muscariniques,

comme produits de combinaison pour une utilisation simultanée, séparée ou échelonnée dans le temps pour le traitement ou la prévention de la dépression, notamment la dépression profonde, la dépression résistante, la dépression du sujet âgé, la dépression psychotique, la dépression induite par des traitements interféron, l'état dépressif, le syndrome maniaco-dépressif, les épisodes dépressifs saisonniers, les épisodes dépressifs liés à une condition médicale générale, les épisodes dépressifs liés à des substances agissant sur l'humeur.

Par psychotrope, on entend désigner une substance d'origine naturelle ou artificielle capable de modifier l'activité mentale et dont l'action essentielle s'exerce sur le système nerveux central et le psychisme. Les psychotropes sont divisés en trois groupes : 1) les psycholeptiques (hypnotiques, neuroleptiques et anxiolytiques), 2) les psychoanaleptiques (antidépresseurs et psychotoniques) et 3) les psychodysleptiques (hallucinogènes).

De préférence, ledit psychotrope est un antidépresseur. A titre d'exemple non limitatif, l'antidépresseur est choisi parmi (i) les inhibiteurs de la mono-amine-oxydase (IMAO) tels que l'iproniazide, la pargyline, la sélègine, (ii) les agonistes 5HT1D tels que le sumatriptan, l'adrénaline et la noradrénaline (sympatho-mimétiques alpha et bêta) (iii) les antidépresseurs tricycliques, tels l'imipramine, la clomipramine, (iv) les inhibiteurs sélectifs de la recapture de la sérotonine (SSRI) tel la fluoxétine, (v) les inhibiteurs sélectifs de la recapture de la norépinéphrine, tels par exemple la tandamine, le fluparoxan, la mirtazapine (vi) les inhibiteurs de la recapture de la sérotonine et de la norépinéphrine, tels la venlafaxine et la duloxétine. A titré d'exemple non limitatif, l'agent anti-muscarinique est sélectionné parmi la toltérodine, la propivérine, l'oxybutynine, le trospium, la darifénacine, la témivérine, l'ipratropium.

De manière préférée, la présente invention a également pour objet l'utilisation comme médicament:

a) ledit énantiomère (1S,2R) du Milnacipran ainsi que ses sels pharmaceutiquement acceptables, et
b) d'au moins un autre principe actif sélectionné parmi les composés actifs induisant une toxicité organique et les composés actifs induisant une toxicité tissulaire, notamment hépatique et/ou rénale ou avec un ou plusieurs principes actifs destinés au traitement de l'insuffisance hépatique et/ou rénale, comme produits de combinaison pour une utilisation simultanée, séparée ou échelonnée dans le temps pour le traitement ou la prévention des affections ou pathologies pouvant être soignées par l'inhibition double de la re-capture de la sérotonine (5-HT) et de la noradrénaline (NA).

De manière préférée, la présente invention a également pour objet l'utilisation comme médicament:

a) ledit énantiomère (1S,2R) du Milnacipran ainsi que ses sels pharmaceutiquement acceptables, et
b) d'au moins un autre principe actif sélectionné parmi les composés actifs induisant des effets secondaires cardiovasculaires et les composés à visée cardiaque,

comme produits de combinaison pour une utilisation simultanée, séparée ou échelonnée dans le temps pour le traitement ou la prévention des affections ou pathologies pouvant être soignées par l'inhibition double de la re-capture de la sérotonine (5-HT) et de la noradrénaline (NA).

Avantageusement, les effets secondaires cardiovasculaires induits sont ceux précédemment évoqués, et plus particulièrement une hypertension artérielle, une hypotension, des troubles du rythme cardiaque (tachycardie, bradycardie, palpitations).

La présente invention a également pour objet les compositions pharmaceutiques contenant les produits de combinaison précédemment décrits.

Dans le cadre de la présente invention, l'énantiomères selon l'invention, de préférence l'énantiomère F2695 substantiellement pur, est avantageusement administré, de façon non limitative, par voie orale, voie nasale, transdermique, rectale, intestinale, parentérale, par injection intramusculaire, sous-cutanée ou intraveineuse, seul ou en association avec d'autres principes actifs, comme précédemment décrit.

Quand ils sont administrés seuls, l'énantiomère selon l'invention, de préférence l'énantiomère F2695 substantiellement pur, peut être administré *per se* ou sous la forme d'une composition pharmaceutique dans laquelle le dit énantiomère ou ses sels pharmaceutiquement acceptables, est en association ou en mélange avec un ou plusieurs supports, excipients et/ou diluants pharmaceutiquement acceptables, facilitant notamment la biodisponibilité.

Quand l'énantiomère selon l'invention, et de préférence l'énantiomère (1S,2R) F2695 du Milnacipran substantiellement pur, est administré en association avec d'autres principes actifs, ledit énantiomère et les autres principes actifs peuvent être formulés en mélange ou à part sous une forme identique ou différente. Ils peuvent être administrés par la même voie ou une voie différente.

Les compositions pharmaceutiques selon l'invention peuvent être formulées de manières conventionnelles bien connues de l'homme de l'art en utilisant un ou plusieurs supports physiologiquement acceptables comprenant des excipients, adjuvants et des auxiliaires tels par exemple des agents conservateurs, des stabilisants, des agents de mouillage ou d'émulsification. La méthode de formulation choisie dépend de la voie d'administration désirée.

Dans le cas d'une administration par injection, on utilise avantageusement une solution aqueuse, notamment une solution tampon physiologiquement acceptable, telle qu'une solution de Hank, une solution de Ringer ou une solution tampon saline physiologique. Dans le cas d'une administration transdermique ou au niveau des muqueuses, on utilise avantageusement des agents pénétrants appropriés à la muqueuse à traverser. De tels agents pénétrants sont bien connus de l'homme du métier. Dans le cas d'une administration orale, les compositions pharmaceutiques selon l'invention sont avantageusement administrées sous formes unitaires ou multidoses d'administration en mélange avec des supports pharmaceutiques adéquats connus de l'homme du métier. Les formes unitaires d'administration appropriées comprennent notamment les comprimés éventuellement sécables, les gélules, les poudres, les granulés et les solutions ou suspensions orales, les aérosols. Les formes multidoses d'administration appropriées comprennent notamment les gouttes buvables, les émulsions et les sirops.

Lors de la préparation de comprimés, l'énantiomère selon l'invention, de préférence l'énantiomère F2695 substantiellement pur, est formulé avec un véhicule pharmaceutiquement acceptable tel que notamment la polyvinylpyrrolidone, le gal carbopol, le polyéthylène glycol, la gélatine, le talc, l'amidon, le lactose, le stéarate de magnésium, la gomme arabique ou leurs analogues. A titre d'exemple, le comprimé contient les excipients suivants : hydrogénophosphate de calcium déshydraté, carmellose calcique, povidone K30, silice colloïdale anhydre, stéarate de magnésium, talc. Les comprimés peuvent éventuellement être enrobés, c'est-à-dire recouverts de plusieurs couches de substances diverses telles que du saccharose, afin de faciliter la prise ou la conservation. L'enrobage peut aussi contenir des pigments ou des colorants afin de distinguer et de caractériser les comprimés en fonction de leur dosage, par exemple. Les comprimés peuvent encore présenter une formulation plus ou moins complexe destinée à modifier la vitesse de libération du principe actif. La libération du principe actif dudit comprimé peut être accéléré, ralentie ou retardée en fonction de l'absorption désirée. L'énantiomère selon l'invention, de préférence l'énantiomère F2695 substantiellement pur, peut être ainsi préparé sous une forme galénique à libération prolongée obtenue selon le procédé décrit dans le brevet EP 939 626. Cette forme galénique se présente sous forme multiparticulaire réunissant une pluralité de minigranules et présente un certain profil de libération *in vitro.*

La libération de l'énantiomère selon l'invention peut être retardée et/ou contrôlée via l'utilisation d'un implant ou via une libération transcutanée, notamment sous-cutanée ou intramusculaire, via une injection intramusculaire ou via un patch transdermique. Ledit énantiomère est alors formulé avec notamment des substances hydrophobes ou polymériques adéquates et des résines échangeuses d'ions.

La quantité à administrer au patient de l'énantiomère selon l'invention, de préférence l'énantiomère F2695 substantiellement pur, dépend des affections à traiter, de l'effet visé, notamment un effet thérapeutique ou prophylactique, de l'état de santé et de l'âge du patient, notamment de ses antécédents cardiovasculaires, des conditions de traitement et du mode d'administration du médicament. Les quantités efficaces thérapeutiques ou prophylactiques à administrer à un patient humain peuvent être déterminées à partir de modèles animaux ou des données, connues de l'homme du métier, lors du traitement de la dépression chez l'homme par exemple par l'utilisation dudit énantiomère de Milnacipran.

Dans le cadre du traitement prophylactique et/ou thérapeutique des pathologies citées ci-dessus, et notamment de la dépression, des états dépressifs, de la fibromyalgie, du syndrome de fatigue chronique, de la douleur, le médicament selon l'invention est avantageusement administré à des doses comprises entre 0,01 mg et 10 mg/kg de poids corporel par jour en une ou plusieurs prises, encore plus avantageusement à des doses comprises entre 0,05 mg et 5 mg / kg de poids corporel par jour en une ou plusieurs prises, encore plus avantageusement à des doses comprises entre 0,1 mg et 1 mg / kg de poids corporel par jour en une ou plusieurs prises. D'une manière particulièrement avantageuse, l'administration dudit médicament à des doses telles que définies ci-dessus est répartie en deux prises quotidiennes, de préférence sous forme de gélule. A titre d'exemple, l'énantiomère selon l'invention, de préférence l'énantiomère F2695 substantiellement pur, est administré sous la forme de gélule dont la teneur en principe actif est avantageusement d'environ 6,75 mg/gélule, 12,5 mg/gélule, 25 mg/gélule, 50 mg/gélule.

D'autres caractéristiques, buts et avantages de l'invention ressortiront des exemples qui suivent. L'invention ne se trouve pas limitée aux exemples particuliers mentionnés à simple titre illustratif et qui doivent être lus en regard des figures suivantes :

<u>Légende des Figures</u>

Figure 1 : Evolution de la fréquence cardiaque après administration unique (valeurs de deltas).
*** $p \leq 0.001$ versus eau désionisée
** $p \leq 0.01$ versus eau désionisée
* $p \leq 0.05$ versus eau désionisée
▲$p \leq 0.05$ versus F2207

Figure 2 : Evolution de la fréquence cardiaque après administration unique (valeurs absolues).
*** $p \leq 0.001$ versus eau désionisée
** $p \leq 0.01$ versus eau désionisée
* $p \leq 0.05$ versus eau désionisée
▲ $p \leq 0.05$ versus F2207

Figure 3 : Effets des divers traitements sur les valeurs moyennes de pressions artérielles diastoliques (moyennes sur les 6 heures suivant le dernier traitement, après 5 jours de traitement consécutifs).

Figure 4 : Effets des divers traitements sur les valeurs moyennes de pressions artérielles systoliques (moyennes sur les 6 heures suivant le dernier traitement, après 5 jours de traitement consécutifs).

Figure 5 : Représentation schématique du mode de calcul de l'index de toxicités. L'index de toxicité est la somme de tous les gènes up et down-régulés (en fonction du facteur d'induction défini par l'utilisateur).

Figures 6a, 6b, 6c : Test au MTT sur des hépatocytes primaires de rats. Les concentrations sont exprimées en μM.

## EXEMPLES

**EXEMPLE N°1:** Etudes pharmacocinétiques du Milnacipran et de ses énantiomères

Des études pharmacocinétiques du chlorhydrate de Milnacipran (F2207) et de ses énantiomères (F2695 et F2696) ont été réalisées dans différentes espèces animale et chez l'homme.

Chez l'animal, la pharmacocinétique de chaque énantiomère a été étudiée suite à l'administration du racémate ou d'un seul énantiomère. Les taux plasmatiques des énantiomères F2695 et F2696 sont approximativement équivalents dans les espèces testées (singe et rat).

Une étude pharmacocinétique chez l'homme comprenant 12 sujets sains a été réalisée en leur administrant le racémate ou un seul des deux énantiomères. Il ressort que le profil pharmacocinétique de chaque énantiomère est indépendant du fait qu'ils aient été administrés soit séparément soit sous forme de racémate indiquant l'absence d'interactions entre les deux énantiomères (**Tableau 1**).

**Tableau 1 : Tableau des principales variables pharmacocinétiques du chlorhydrate de Milnacipran (F2207) et de ses deux énantiomères F2695 et F2696.**

Cmax : Concentration plasmatique maximale estimée directement à partir des données expérimentales
Tmax : Temps d'obtention de la concentration plasmatique maximale
$AUC_{0\rightarrow\infty}$ : Aire sous la courbe des concentrations plasmatiques en fonction du temps extrapolé à l'infini
$T_{1/2}$ : Demi-vie terminale de décroissance des concentration plasmatiques

| **Dose administrée (mg)** | **F2207 (50 mg)** | | **F2695 (D) (25 mg)** | **F2696 (L) (25 mg)** |
|---|---|---|---|---|
| | **F2695 (D)** | **F2696 (L)** | | |
| **Cmax (nmol.l$^{-1}$)** | **214** | **179** | **216** | **212** |
| **Tmax (heure)** | **3,42** | **2,87** | **3,08** | **2,21** |
| **AUC 0->°° (nmol.h.l$^{-1}$)** | **2896** | **1563** | **2869** | **1543** |
| **T ½ (heures)** | **9,28** | **5,75** | **9,38** | **5,58** |

Ces résultats indiquent qu'aucune bioconversion des énantiomères F2695 et F2696) n'a été détectée dans les espèces analysées.

**EXEMPLE N°2** : Etudes biochimiques du Milnacipran et de ses énantiomères

Les deux énantiomères (F2695 et F2696) du chlorhydrate de milnacipran (F2207) ont été étudiés *in vitro* sur les captures de noradrénaline et sérotonine ainsi que sur la liaison (binding) de la paroxétine au niveau du cerveau du rat.

### 2.1. MATERIELS ET METHODES

#### 2.1.1. Capture de noradrénaline par un homogénat ($P_2$) d'hypothalamus de rat.

Préparation du P2

Des rats mâles, Sprague-Dawley, de 200 à 300 g sont assommés et décapités, puis l'hypothalamus prélevé rapidement. Deux hypothalami sont homogénéisés dans 4 ml de sucrose 0,32 M au Potter S par 16 allers et retours complets à 800 tours/min, puis centrifugés 10 mn à 1 000 g pour éliminer les débris cellulaires. Le surnageant est centrifugé 20 mn à 10 000 g et le $P_2$ ainsi obtenu repris dans 4 ml de sucrose 0,32 M et homogénéisé au Dounce.

Capture (« Uptake »)

On utilise de la $^3H$-(1)-NA : 13 Ci/mmole (Amersham).

L'uptake se fait en tampon phosphate (contenant par litre : 8 g de NaCl, 1,21 g de $K_2HPO_4$ et 0,34 g de $KH_2PO_4$) préoxygéné 30 mn avant utilisation par un mélange $O_2/CO_2$ (95 % / 5 %).

Dans des tubes en plastique de 5 ml placés au bain marie à 37°C sont introduits :

- 100 µl de tampon ou inhibiteur,
- 700 µl de tampon (contenant de la pargyline 25 µM),
- 100 µl de $P_2$.

Après équilibration de la température, la réaction démarre par addition de 100 µl de $^3H$-NA, 50 nM en final.

10 mn exactement après, la réaction est stoppée par addition de 2,5 ml de tampon glacé et filtration sur filtres GF/F. Puis le tube est rincé une fois et le filtre une fois avec 2,5 ml de tampon glacé. Le filtre est ensuite introduit dans une mini-fiole Beckman et après addition de 3 ml de liquide scintillant Instagel (Packard), la radioactivité est mesurée dans un compteur à scintillation liquide Tricarb Packard.

L'uptake non spécifique (NS) est mesuré en présence de DMI $10^{-5}$ M.

Le pourcentage d'inhibition est calculé par la formule :

$$\frac{(\text{uptake total} - \text{NS}) - (\text{uptake en présence d'inhibiteur} - \text{NS})}{(\text{uptake total} - \text{NS})}$$

L'$IC_{50}$ est déterminée graphiquement sur la courbe moyenne des pourcentages d'inhibition (4 essais) en fonction du log de la concentration en inhibiteur.

**2.1.2. Capture de sérotonine**

La méthode a été réalisée d'après Gray et Whittaker (1962, J. Anat., 96 : 79-97). Après homogénéisation du tissu cérébral dans une solution de sucrose, les terminaisons présynaptiques se détachent de l'axone et se referment pour former des synaptosomes obtenus par fractionnement subcellulaire.

Des rats mâles Sprague-Dawley (Janvier) de 180-200 g ont été utilisés. Après sacrifice de l'animal, l'hypothalamus a été prélevé, pesé, homogénéisé au Dounce dans du sucrose 0,32 M à 0°C.

Cet homogénat a été centrifugé 10 mn à 1 000 g (2 400 tours/mn - Hettich, Rotenta). Le surnageant a été récupéré et centrifugé 20 mn à 10 000 g (8 000 tours/mn - Beckam, modèle J2-21 M : rotor J14). Le culot (appelé fraction $P_2$) a été repris dans du sucrose selon une concentration de 50 mg/ml.

On a incubé pendant 5 mn à 37°C :

o 350 µl de tampon glacé (NaCl 136mM, $KH_2PO_4$ 2,4mM, $K_2HPO_4$ 6,9 mM, pH 7,2) préoxygéné 30 mn avant,
o 50 µl de membranes (5 mg/ml final),
o 50 µl de citalopram ($10^{-5}$ M final) pour la capture non spécifique,
o 50 µl de $^3H$-5-HT (50 nM final) (NEN, France, 28,4 Ci/mmol).

Exactement 5 mn après le début de l'incubation, la réaction a été arrêtée par filtration sous vide sur filtres Whatman GF/F (prédilution avec 2,5 ml de tampon glacé puis rinçage avec 3 fois 2,5 ml).

La radioactivité recueillie sur le filtre a été mesurée (Packard Tricarb 4640) par scintillation liquide avec Emulsifier-Safe (Packard).

Les $IC_{50}$ ont été déterminées en portant graphiquement les pourcentages d'inhibition en fonction du log de la concentration en produit (6 concentrations en duplicate).

**2.1.3. Liaison (« Binding ») de la paroxétine**

Des rats mâles Sprague-Dawley (Janvier) de 180-200 g ont été utilisés. Les hypothalami de plusieurs rats ont été rassemblés et homogénéisés dans 5 ml de tampon glacé (50 mM Tris-HCL, 120 mM NaCl, 5 mM KCl, pH 7,5) au Dounce, et l'homogénat centrifugé à 30 000 g (27 000 t/mn - Beckman . L5-50E, rotor T40) pendant 10 mn. Le culot obtenu a été repris dans 5 ml de tampon et recentrifugé dans les mêmes conditions. Le nouveau culot a été repris dans le même tampon et finalement réhomogénéisé au Dounce à une concentration tissulaire de 10 mg/ml. La suspension

membranaire (100 µl) a été incubée avec la 3H-paroxétine (NEN, France, 28,6 Ci/mmol) à la concentration (finale) de 0,1 nM, à 20°C, dans un volume final de 1 ml pendant 2 h. Au bout des 2 h d'incubation, la réaction a été arrêtée par filtration sous vide sur filtres Whatman GF/F prétraités dans une solution de polyéthylénimine à 0,05 % 30 mn auparavant (prédilution avec 4 ml de tampon glacé puis rinçage du tube avec 2 fois 4 ml). La radioactivité a été mesurée par spectrométrie de scintillation liquide (Packard, Tricarb 4640) en utilisant l'Emulsifier-Safe (Packard) comme agent scintillant.

Le binding spécifique de la $^{3}H$-paroxétine a été défmi comme la différence entre le binding total et celui restant en présence de 10 µM de fluoxétine.

Les $IC_{50}$ ont été déterminées en portant graphiquement les pourcentages d'inhibition en fonction du log de la concentration en produit (6 concentrations en duplicate).

**2.1.4. Produits utilisés**

F2207 : lot n° 10-CTN3 Clé P118
F2695 : lot n° PL-I-205
F2696 : lot n° PL-I-204C.

**2.2. RESULTATS**

Les effets du F2207 et de ses deux énantiomères sur la capture de noradrénaline et sérotonine et sur le binding de la paroxétine sont représentés sur un graphe avec en ordonnée le pourcentage d'inhibition en fonction (%) et en abscisse la concentration (M) du F2207, F2695 ou du F2696 (données non montrées). Les valeurs des pourcentages d'inhibition correspondant à chaque concentration de produit, testée en duplicate, sont les moyennes des résultats de quatre expériences indépendantes.

Les valeurs des $IC_{50}$ des trois produits ont été déterminées à partir de ces courbes et figurent dans le tableau 2.

**Tableau 2 : Inhibition des captures de $^{3}H$-noradrénaline, $^{3}H$-sérotonine et liaison (binding) de la $^{3}H$-paroxétine.**

| IC50 (M) | | | |
|---|---|---|---|
| Composés | Capture | | Liaison (binding) $^{3}H$-Paroxétine |
| | $^{3}H$-Noradrénaline | $^{3}H$-Sérotonine | |
| **F2695** | 1,5 X $10^{-8}$ | 4,6 X $10^{-8}$ | 6,0 X $10^{-8}$ |
| **F2207** | 3,0 X $10^{-8}$ | 15X$10^{-8}$ | 13 X $10^{-8}$ |
| **F2696** | 75 X $10^{-8}$ | 60X$10^{-8}$ | 70 X $10^{-8}$ |

Les trois composés sont actifs sur ces trois tests pharmacologiques, mais des différences existent :

- sur la capture de noradrénaline :
  Le F2695 est 2 fois plus actif que le F2207.
  Le F2695 est 25 fois plus actif que le F2696.
- sur la capture de sérotonine :
  Le F2695 est 3 fois plus actif que le F2207.
  Le F2695 est 12 fois plus actif que le F2696.
- sur la liaison (binding) de la paroxétine :
  Le F2695 est 2 fois plus actif que le F2207.
  Le F2695 est 10 fois plus actif que le F2696.

Les trois composés sont actifs sur ces tests pharmacologiques avec cependant une moindre activité pour la forme (1R,2S) (F2696) et le racémate (F2207). La forme (1S,2R) du chlorhydrate de Milnacipran (F2695) est 2 à 3 fois plus active que le F2207.

**EXEMPLE N°3** : Activité comparative par voie orale du chlorhydrate de Milnacipran racémique (F2207) et de son énantiomère (1S, 2R) actif (F2695) sur la fréquence cardiaque et la pression artérielle chez le chien vigile.

3.1. **INTRODUCTION**

Cette étude se propose d'étudier les effets du F2207 et du F2695 a) en administration unique par voie orale sur la fréquence cardiaque (n = 28 chiens), et b) après traitement réitéré de 5 jours par voie orale sur les pressions artérielles systolique et diastolique chez le chien (n = 6 chiens).

Cette étude a été menée à équidoses pharmacologiquement actives de F2207 et F2695 sur des animaux femelles équipés d'implants (Data Sciences International) permettant l'acquisition de la fréquence cardiaque et des paramètres tensionnels par télémétrie. Ces animaux ont été répartis pour toutes les études en 3 groupes de traitement :

- groupe 1 (témoin) traité à l'eau désionisée,
- groupe 2 traité au F2207 à la dose de 20 mg/kg/J,
- groupe 3 traité au F2695 à la dose de 10 mg/kg/J.

**3.2. METHODOLOGIE**

Compte tenu du faible nombre d'animaux simultanément équipés (maximum 8), du nombre de pistes d'enregistrement de l'équipement utilisé (8 pistes), et de sorte à constituer des groupes de traitement homogènes, l'évaluation globale a été réalisée en quatre études, chaque étude étant partitionnée en trois passages (traitement de chaque animal avec chacun des trois produits), séparés par une période de lavage et de ré-initialisation de sondes (« wash-out »). Chaque passage lui-même se déroule en deux phases :

- une première phase durant laquelle tous les animaux sont traités à l'eau désionisée pour une habituation à la contention et au traitement oral par sonde de gavage,
- une deuxième phase durant laquelle les animaux reçoivent leur traitement respectif (administration unique pour la fréquence cardiaque, études n° 894 / 926 / 935 / 936 ; administration réitérée cinq jours pour la pression artérielle, étude n° 894).

Le schéma expérimental global est décrit dans le tableau suivant :

**Tableau 3 : Schéma expérimental global pour l'étude par télémétrie des effets du chlorhydrate de Milnacipran racémique (F2207) et de son énantiomère (1S, 2R) actif F2695 administrés oralement chez le chien conscient.**

| GROUPE NUMERO | 1 | 2 | 3 |
|---|---|---|---|
| ANIMAUX | | | |
| Nombre | 27 | 28 | 28 |
| Identification | 1-2-7-8-13-14 (étude 894)<br>1-2-7-8-13-14 (étude 926)<br>1-2-9-10 11-17-18-19 (étude 935)<br>1-2-9-10 11-17-18-19 (étude 936) | 3-4-9-10-15-16 (étude 894)<br>3-4-9-10-15-16 (étude 926)<br>3-4-5-12 13-20-21-22 (étude 935)<br>3-4-5-12 13-20-21-22 (étude 936) | 5-6-11-12-17-18 (étude 894)<br>5-6-11-12-17-18 (étude 926)<br>6-7-8-14 15-16-23-24 (étude 935)<br>6-7-8-14 15-16-23-24 (étude 936) |
| TRAITEMENT | | | |
| Identification | Eau désionisée | F2207 | F2695 |
| Dose | | 20 mg/kg | 10 mg/kg |
| Voie | | orale | |
| Volume | | 5 ml/kg | |
| *(n = 27 dans le groupe témoin, le signal de sonde de l'animal n° 18 n'ayant pas été enregistré)* | | | |

Les effets des différents traitements sur la fréquence cardiaque ont été analysés dans les quatre études, après administration unique. L'analyse a porté sur les 13 temps d'acquisition suivants :

- avant traitement unique,
- toutes les 30 minutes suivant les 6 heures après traitement unique.

Les effets des différents traitements sur la pression artérielle ont été analysés, dans l'étude n° 894 à l'état d'équilibre, à J5, J29 et J33 (dernier jour de traitement effectif pour chacun des passages). L'analyse a porté sur les temps d'acquisition suivants :

- avant traitement,
- toutes les 30 minutes suivant les 6 heures après traitement.

### 3.3. RESULTATS

3.3.1. En ce qui concerne la fréquence cardiaque (quatre études poolées), un test de Tukey a été réalisé sur les deltas de fréquence individuels, pour chacun des 12 temps expérimentaux post-traitement, versus la valeur pré-traitement, ainsi que sur les valeurs absolues de fréquence cardiaque à chaque temps d'enregistrement.

Ont été ainsi objectivées, par rapport aux animaux témoins recevant l'eau désionisée : # lorsque l'analyse statistique est réalisée sur les valeurs de deltas, (figure 1) :

- une augmentation significative de la fréquence cardiaque dès la première ½ heure suivant administration unique de F2207 (20 mg/kg), augmentation persistant jusqu'à 5.5 heures après traitement ($p \leq 0.001$ pour l'ensemble des temps d'acquisition, à l'exception des temps 0.5 et 5.5 heures - $p \leq 0.01$ - et du temps 5.0 heures - $p \leq 0.05$ - après traitement),
- une augmentation de la fréquence cardiaque après l'administration du F2695 qui reste toujours inférieure à celle obtenue après administration de F2207. De plus, cette différence entre les effets du F2207 et du F2695 est significative ($p<0.05$) à 1 et 4h après administration en faveur du F2695.
- une augmentation de la fréquence cardiaque qui dure moins longtemps sous F2695 (1.0 à 4.5 h) que sous F2207 (persiste jusqu'à 5.5h après traitement).

# lorsque l'analyse statistique est réalisée sur des valeurs absolues de fréquence cardiaque, cette même étude met en évidence (figure 2) :

- une augmentation significative de la fréquence cardiaque dès la première heure suivant administration unique de F2207 (20 mg/kg), augmentation persistant jusqu'à 5.5 heures après traitement ($p \leq 0.001$ pour l'ensemble des temps d'acquisition de 1.0 à 4.5 heures, à l'exception du temps 3.5 heures - $p \leq 0.01$ ; et $p \leq 0.01$ pour le temps d'acquisition 5.5 heures après traitement),
- une augmentation de la fréquence cardiaque après l'administration du F2695 qui reste toujours inférieure à celle obtenue après administration de F2207. De plus, cette différence entre les effets du F2207 et du F2695 est significative ($p<0.05$) à 1 et 4h après administration en faveur du F2695.
- une augmentation de la fréquence cardiaque qui dure moins longtemps sous F2695 (1.0 à 4.5 h) que sous F2207 (persiste jusqu'à 5.5h après traitement).

3.3.2. En ce qui concerne la pression artérielle (une étude en administration réitérée), une valeur moyenne de pression artérielle diastolique (figure 3 et tableau 4), ainsi qu'une valeur moyenne de pression artérielle systolique (figure 4 et tableau 5) ont été calculées pour chaque chien et pour les 6 heures suivant le dernier traitement, après 5 jours consécutifs d'administration. Ces valeurs moyennes de pressions ont été analysées par une ANOVA suivie d'un test de Tukey lorsque cette dernière le permettait (données non montrées).

Ont ainsi été objectivés :

- une augmentation significative ($p \leq 0.001$) de la pression artérielle diastolique après administration réitérée 5 jours de F2207 (20 mg/kg/J) ou de F2695 (10 mg/kg/J) comparativement au traitement à l'eau désionisée,
- une différence significative ($p \leq 0.05$) de la valeur de pression artérielle diastolique moyennée suivant administration réitérée 5 jours de F2207 (20 mg/kg/J) comparativement à la valeur moyennée de pression artérielle diastolique suivant l'administration réitérée de F2695 (10 mg/kg/J),
- aucun effet significatif au niveau de la pression artérielle systolique ; on peut toutefois remarquer que les valeurs de PAS après administration réitérée 5 jours de F2695 sont proches des valeurs de PAS suivant traitement à l'eau

désionisée.

Les données individuelles de pressions artérielles diastolique et systolique sont présentées dans les tableaux 4 et 5 respectivement.

**Tableau 4 : Données individuelles de pression artérielle diastolique**

**PRESSION ARTERIELLE DIASTOLIQUE (PAD exprimée en mm Hg)**
Données individuelles après administration réitérée 5 jours consécutifs

| GROUPE | 1 | | | | | | | | 2 | | | | | | | | 3 | | | | | | | |
|---|---|---|---|---|---|---|---|---|---|---|---|---|---|---|---|---|---|---|---|---|---|---|---|---|
| TRAITEMENT | VEHICULE | | | | | | | | F2207 (20 mg/kg/J) | | | | | | | | F2695 (10 mg/kg/J) | | | | | | | |
| Animal N° | 1 | 2 | 7 | 8 | 13 | 14 | Moy. | e.s.m. | 3 | 4 | 9 | 10 | 15 | 16 | Moy. | e.s.m. | 5 | 6 | 11 | 12 | 17 | 18 | Moy. | e.s.m. |
| Tps avant traitement | 79 | 77 | 73 | 77 | 101 | 76 | 81 | 4 | 112 | 89 | 93 | 88 | 86 | 91 | 93 | 4 | 73 | 89 | 80 | 71 | 76 | 78 | 78 | 3 |
| Tps après traitement (h) | | | | | | | | | | | | | | | | | | | | | | | | |
| 0.50 | 84 | 76 | 70 | 63 | 80 | 70 | 74 | 3 | 103 | 106 | 96 | 92 | 88 | 87 | 95 | 3 | 91 | 91 | 99 | 90 | 108 | 85 | 94 | 3 |
| 1.00 | 82 | 84 | 77 | 72 | 72 | 76 | 77 | 2 | 130 | 117 | 113 | 113 | 90 | 106 | 112 | 5 | 112 | 96 | 75 | 97 | 87 | 96 | 94 | 5 |
| 1.50 | 102 | 81 | 79 | 75 | 82 | 68 | 81 | 5 | 131 | 127 | 137 | 96 | 100 | 91 | 114 | 8 | 109 | 83 | 88 | 97 | 87 | 112 | 96 | 5 |
| 2.00 | 83 | 75 | 71 | 98 | 77 | 75 | 80 | 4 | 123 | 113 | 99 | 88 | 107 | 109 | 107 | 5 | 115 | 88 | 93 | 95 | 84 | 109 | 97 | 5 |
| 2.50 | 85 | 75 | 75 | 84 | 85 | 79 | 81 | 2 | 137 | 111 | 116 | 101 | 115 | 107 | 115 | 5 | 111 | 88 | 97 | 89 | 92 | 107 | 97 | 4 |
| 3.00 | 91 | 95 | 99 | 85 | 79 | 84 | 89 | 3 | 121 | 118 | 112 | 116 | 106 | 92 | 111 | 4 | 104 | 91 | 96 | 96 | 100 | 106 | 99 | 2 |
| 3.50 | 83 | 72 | 78 | 73 | 77 | 65 | 75 | 3 | 120 | 106 | 133 | 116 | 103 | 103 | 114 | 5 | 96 | 106 | 94 | 107 | 77 | 103 | 97 | 5 |
| 4.00 | 81 | 79 | 75 | 77 | 82 | 68 | 77 | 2 | 133 | 114 | 105 | 111 | 110 | 103 | 113 | 4 | 125 | 91 | 99 | 108 | 80 | 109 | 102 | 6 |
| 4.50 | 82 | 76 | 91 | 84 | 113 | 85 | 89 | 5 | 135 | 110 | 126 | 109 | 104 | 108 | 115 | 5 | 103 | 104 | 92 | 100 | 85 | 108 | 99 | 3 |
| 5.00 | 97 | 79 | 67 | 95 | 81 | 82 | 84 | 5 | 116 | 120 | 98 | 97 | 97 | 105 | 106 | 4 | 126 | 100 | 92 | 95 | 110 | 102 | 104 | 5 |
| 5.50 | 94 | 80 | 70 | ND | 85 | 82 | 82 | 4 | 103 | 107 | 115 | 106 | 92 | 93 | 103 | 4 | 88 | 86 | 105 | 98 | 89 | 99 | 94 | 3 |
| 6.00 | 83 | 74 | 82 | 82 | 78 | 77 | 79 | 1 | 115 | 133 | 120 | 104 | 103 | 104 | 113 | 5 | 101 | 113 | 98 | 105 | 109 | 108 | 106 | 2 |
| **PAD moyenne après traitement** | **87** | **79** | **78** | **81** | **83** | **76** | **81** | **2** | **122** | **115** | **114** | **104** | **101** | **101** | **110** | **4** | **107** | **95** | **94** | **98** | **92** | **104** | **98** | **2** |

ND : non déterminé

## Tableau 5 : Données individuelles de pression artérielle systolique

**PRESSION ARTERIELLE SYSTOLIQUE (PAS exprimée en mm Hg)**

Données individuelles après administration réitérée 5 jours consécutifs

| GROUPE | 1 | | | | | | | | 2 | | | | | | | | 3 | | | | | | | |
|---|---|---|---|---|---|---|---|---|---|---|---|---|---|---|---|---|---|---|---|---|---|---|---|---|
| TRAITEMENT | VEHICULE | | | | | | | | F2207 (20 mg/kg/J) | | | | | | | | F2695 (10 mg/kg/J) | | | | | | | |
| Animal N° | 1 | 2 | 7 | 8 | 13 | 14 | Moy. | e.s.m. | 3 | 4 | 9 | 10 | 15 | 16 | Moy. | e.s.m. | 5 | 6 | 11 | 12 | 17 | 18 | Moy. | e.s.m. |
| Tps avant traitement | 139 | 141 | 120 | 157 | 172 | 138 | 145 | 7 | 188 | 164 | 176 | 149 | 130 | 169 | 163 | 8 | 136 | 141 | 138 | 130 | 134 | 149 | 138 | 3 |
| Tps après traitement (h) | | | | | | | | | | | | | | | | | | | | | | | | |
| 0.50 | 135 | 132 | 119 | 131 | 149 | 138 | 134 | 4 | 158 | 154 | 152 | 128 | 126 | 129 | 141 | 6 | 135 | 129 | 140 | 135 | 160 | 139 | 140 | 4 |
| 1.00 | 134 | 158 | 129 | 144 | 141 | 143 | 142 | 4 | 180 | 167 | 157 | 150 | 126 | 130 | 152 | 9 | 159 | 135 | 124 | 148 | 131 | 143 | 140 | 5 |
| 1.50 | 158 | 151 | 145 | 150 | 153 | 137 | 149 | 3 | 186 | 181 | 189 | 129 | 136 | 138 | 160 | 12 | 164 | 119 | 138 | 158 | 127 | 156 | 144 | 8 |
| 2.00 | 138 | 136 | 145 | 173 | 151 | 144 | 148 | 5 | 171 | 160 | 146 | 122 | 140 | 163 | 150 | 7 | 168 | 125 | 135 | 141 | 127 | 156 | 142 | 7 |
| 2.50 | 142 | 143 | 145 | 159 | 160 | 148 | 150 | 3 | 195 | 168 | 168 | 144 | 153 | 161 | 165 | 7 | 165 | 124 | 142 | 141 | 134 | 154 | 143 | 6 |
| 3.00 | 149 | 167 | 162 | 163 | 150 | 154 | 158 | 3 | 173 | 177 | 164 | 157 | 141 | 146 | 160 | 6 | 156 | 131 | 145 | 144 | *151* | 157 | 147 | 4 |
| 3.50 | 135 | 129 | 149 | 154 | 153 | 137 | 143 | 4 | 165 | 153 | 184 | 167 | 139 | 155 | 161 | 6 | 146 | 147 | 141 | 169 | 123 | 156 | 147 | 6 |
| 4.00 | 142 | 143 | 149 | 166 | 164 | 144 | 151 | 4 | 180 | 157 | 151 | 154 | 150 | 153 | 158 | 5 | 180 | 132 | 145 | 160 | 124 | 164 | 151 | 9 |
| 4.50 | 137 | 140 | 159 | 170 | 190 | 152 | 158 | 8 | 184 | 161 | 180 | 155 | 145 | 168 | 166 | 6 | 158 | 151 | 138 | 163 | 131 | 163 | 151 | 5 |
| 5.00 | 150 | 146 | 127 | 177 | 160 | 145 | 151 | 7 | 161 | 171 | 146 | 141 | 139 | 166 | 154 | 6 | 182 | 144 | 137 | 150 | 162 | 158 | 156 | 6 |
| 5.50 | 153 | 149 | 132 | ND | 148 | 144 | 145 | 4 | 151 | 154 | 173 | 152 | 132 | 155 | 153 | 5 | 142 | 127 | 152 | 153 | 141 | 153 | 145 | 4 |
| 6.00 | 146 | 144 | 151 | 176 | 146 | 143 | 151 | 5 | 158 | 192 | 171 | 154 | 148 | 172 | 166 | 7 | 156 | 170 | 148 | 159 | 160 | 166 | 160 | 3 |
| **PAS moyenne après traitement** | **143** | **145** | **143** | **160** | **155** | **144** | **148** | **3** | **172** | **166** | **165** | **146** | **140** | **153** | **157** | **5** | **159** | **136** | **140** | **152** | **139** | **155** | **147** | **4** |

ND : non déterminé

### 3.4. CONCLUSION

Dans les conditions expérimentales de la présente évaluation, menée en quatre études successives par administration orale chez le chien vigile appareillé en télémétrie :

¤ en administration unique et comparativement au groupe témoin (n = 28), l'augmentation de la fréquence cardiaque est nettement significative et durable avec le F2207 à la dose de 20 mg/kg/J ; elle est statistiquement et cliniquement moindre et plus fugace avec le F2695 à la dose pharmacologiquement équiactive de 10 mg/kg/J,
¤ le F2695, à la dose de 10 mg/kg/J, ne provoque aucune modification statistiquement significative de la pression artérielle systolique moyennée sur les 6 heures suivant le dernier traitement, à l'état d'équilibre après administration réitérée 5 jours,
¤ une différence statistiquement significative est mise en évidence sur les valeurs de la pression artérielle diastolique moyennée sur les 6 heures suivant le dernier traitement, à l'état d'équilibre après administration réitérée 5 jours, entre l'énantiomère actif F2695 (98 $\pm$ 2 mm Hg) et le racémique F2207 à équidoses pharmacologiquement actives (110 $\pm$ 4 mm Hg).

Ces différences témoignent clairement d'une meilleure tolérance cardiovasculaire de l'énantiomère actif F2695.

## EXEMPLE 4 : TEST de PREDICTIVITE PHARMACO-TOXICO-GENOMIQUE *IN VITRO.*

### 4.1. MATERIELS ET METHODES

Les composés F2695 et F2696, énantiomères de la molécule racémique F2207, ainsi qu'un produit de référence, la clomipramine (codée dans l'essai C218) ont été évalués au cours de la présente étude. Les deux énantiomères F2695 et F2696 ont été tout d'abord évalués dans un test préliminaire de cytotoxicité (test au MTT) sur des hépatocytes primaires de rat, de façon à sélectionner les trois concentrations à utiliser dans le test définitif.
Après traitement d'hépatocytes primaires de rat en culture, l'ARN a été extrait de façon à générer des sondes d'ADN complémentaire marquées, qui ont été ensuite hybridées sur une membrane contenant 682 fragments d'épissage alternatif spécifiques du stress cellulaire. Des index de toxicité ont été obtenus, pour chaque produit testé, en comparant le profil d'hybridation des cellules traitées avec celui obtenu à partir de cellules non traitées.

#### 4.1.1. Principe et but de l'étude

Safe-Hit est un test de pharmaco-toxicogénomique prédictif sensible, robuste, fiable, rapide et sûr qui permet la comparaison et le classement de produits, basé sur l'évaluation optimisée de leur potentiel toxique.
Safe-Hit bénéficie d'une technologie, propriété de EXONHIT (DATAS™ : *Differential Analysis of Transcripts with Alternative Splicing),* permettant d'isoler et, par conséquent, de cloner les évènements d'épissage résultant d'un état biologique donné, comparativement à une condition contrôle. Cela permet l'isolement d'isoformes d'ARNm, exprimées différemment en fonction de la condition biologique.
Safe-Hit permet le classement des molécules au sein d'une série chimique, en fonction d'un Index Toxique, déterminé après les étapes de base suivantes (systématiquement mises en oeuvre en duplicate pour chaque produit) :

- traitement des lignées cellulaires avec les différents produits, à trois concentrations déduites d'un test préalable de toxicologie cellulaire (test MTT) : une concentration de référence correspondant à 80% de viabilité cellulaire, une concentration 10 fois supérieure - quand cela est possible - et une concentration 10 fois inférieure,
- préparation de l'ARN total et des sondes radiomarquées de cADN correspondantes,
- hybridation des sondes de cADN : Safe-Hit macro-array contenant 682 clones indépendants, correspondant à des modifications d'épissage induites par la surexpression de WTp53 (p53 est le « médiateur » le plus ubiquitaire du stress cellulaire choisi pour le développement de cette méthodologie),
- acquisition et détermination de l'Index de Toxicité.

#### 4.1.2. Cellules

Les cellules utilisées pour l'étude (test MTT préalable de cytotoxicité et test principal) sont des hépatocytes cryopréservés de rat Sprague-Dawley en primoculture (lots Hep184005 et Hep184006 - Biopredic), cultivés en conditions standard.

*4.1.2.1 Milieux de culture*

- *milieu de décongélation :* milieu de Leibovitz 15 avec glutamax 1, additionné de 100 IU/ml de pénicilline, 100 μg/ml de streptomycine et 0,6 M de glucose (lot MIL 210009 -Biopredic),
- *milieu d'ensemencement :* milieu de Williams E avec glutamax 1, additionné de 100 IU/ml de pénicilline, 100 μg/ml de streptomycine, 4 μg/ml d'insuline bovine et 10 % v/v de sérum de veau foetal (lot MIL 260005) - Biopredic),
- *milieu d'incubation :* milieu de Williams E avec glutamax 1, additionné de 100 IU/ml de pénicilline, 100 μg/ml de streptomycine, 4 μg/ml d'insuline bovine et 50 μM d'hémisuccinate d'hydrocortisone (lot MIL 260009-260007 - Biopredic).

*4.1.2.2 Conditions de culture*

37°C, atmosphère CO2 (5%), hygrométrie relative (95%).

*4.1.2.3 Procédé de culture*

| | Test de toxicité cellulaire | Etude principale |
|---|---|---|
| | Cellules ensemencées le jour du traitement | |
| Densité d'ensemencement | 35 000 cellules / puits (plaque 96 puits) | 1,5 million de cellules par plaque de 30 mm |
| Volume de milieu | 0,1 ml | 3 ml |

**4.1.3. Test de cytotoxicité**

Ce test de cytotoxicité (test MTT) détecte les cellules vivantes grâce à une réaction colorimétrique qui révèle l'intégrité de la respiration cellulaire impliquant l'activité des mitochondries. Le MTT (3-[4,5-dimethylthiazol-2-yl]-2,5-diphenyltetrazolium bromide), soluble dans l'eau, est transformé par clivage, sous l'action d'une enzyme mitochondriale des cellules vivantes, en un formazan pourpre insoluble. Le formazan est solubilisé dans un solvant organique et la solution obtenue peut être mesurée par spectrophotométrie. L'absorbance mesurée est proportionnelle au nombre de cellules survivantes.

Les cellules sont mises en contact pendant 16 heures avec le produit à tester à 5 concentrations différentes (0 - 1 - 10 - 25 - 50 et 100 μM).

Après cette phase d'exposition, une solution de MTT (0,5 mg/ml dans le milieu d'incubation des hépatocytes primaires) est ajoutée pendant 3 heures. Après solubilisation des cristaux de formazan, les plaques multi puits sont lues par un spectrophotomètre à 500 nm afin de déterminer le pourcentage de viabilité cellulaire.

**4.1.4 Etude principale de pharmaco-toxicogénomique**

L'étude principale est réalisée en duplicate, à partir des cultures ensemencées et exposées à chaque produit, afin d'accroître la cohérence entre les expériences et valider les résultats obtenus.

*4.1.4.1 Ensemencements cellulaires et traitement*

Les cellules sont ensemencées et cultivées pendant 16 heures avec chaque produit, aux trois concentrations choisies à partir du test préalable MTT ; deux contrôles (cellules non traitées, solvant seul) sont ajoutés pour la série.

*4.1.4.2 Extraction de l'ARN total et dosage*

Après traitement, l'ARN est extrait et analysé comme suit :

- recueil des cellules et centrifugation,
- extraction réalisée avec un réactif phénol prêt-à l'emploi (Trizol - lot 1106266 et 1121067 - Invitrogen) suivant le

protocole du fabricant,
- solubilisation de l'ARN dans l'eau,
- dosage de l'ARN en spectrophotométrie (densité optique mesurée à 260, 280 et 300 nm),
- vérification de la qualité de l'ARN par Agilent.

***4.1.4.3 Préparation des sondes d'ADNc***

Les sondes d'ADNc sont préparées par transcription inversée radioactive (alpha dATP $^{33}P$ - Amersham). La quantification d'ADNc radioactif (Instant Imager - Packard) est réalisée pour confirmer l'activité des sondes.

***4.1.4.4 Hybridation sur membrane Safe-Hit***

Sur les membranes Safe-Hit en nylon prédécoupé (Q-BIOgene) sont déposés en double 682 clones DATAS (motifs d'épissage alternatif), à l'aide d'un appareillage de Q-Pix (GENETIX). Les sondes d'ADN sont hybridées durant la nuit sur les membranes, puis celles-ci sont lavées.

***4.1.1.5. Préparation des sondes d'ADNc :***

- matrice : 5 μg d'ARN total (pour chaque série de traitement et pour chaque concentration),
- amorce : 100 ng d'oligonucléotide oligo-dTV, pour la 1ère et 2ème hybridation sur le rat (lot 12.00, Invitrogen),
- mélange principal :

  10 μl de tampon First Strand 5x Premier (lot 1131226 - Invitrogen)
  1 μl dCTP+dGTP+dTTP 20 mM (lot 1105201 - Invitrogen)
  1 μM d'ATP 120 μM (lot 1105291 - Invitrogen)
  5 μl Dithiotréitol (DTT) 0,1 M (lot 133609 - Invitrogen)
  1 μl RNase Out 40 U (lot 1113345 - Invitrogen)
  5 μl d'$\alpha$ $^{33}P$ dATP 3 000Ci/mmol 10 mCi/μl (lot B0239 - Amersham)
  4 μl Superscript II (lot 1137806 - Invitrogen)
  1 μl de glycogène (lot 1129328 - Invitrogen)

**- *procédure :***

incuber l'ARN et l'oligo-dTV à 70°C pendant 10 minutes puis mettre dans la glace. Ajouter 27 μl de MasterMix puis incuber à 43°C / 1h puis à 50°C / 15 minutes. Ajouter 20 μl d'eau, puis 20 μl d'EDTA 50 mM, puis 4 μl de NaOH ION. Incuber 20 minutes à 65°C puis mettre dans la glace.
Quantification : Instant Imager, Packard : 1 μl de mélange réactionnel, ajouter 8 μl d'acide acétique, 100 μl d'isopropanol et 1 μl de glycogène (20 μg/μl). Incuber à -20°C pendant 20 minutes, centrifuger 20 minutes à 13000 t/mn à 4°C. Remettre en suspension dans 200μl d'eau,
quantification : Instant Imager, Packard : 1 μl de mélange réactionnel.

***- Milieux et tampons***

| *Solutions communes :* | *Tampon de lavage 1 :* |
|---|---|
| 20X SSC (Invitrogen) | 2X SSC |
| 50X Denhardt's | |
| 50 % (w/v) Sulfate de Dextran (ICN) | |
| 20 % SDS (v/v)(Quantum biotech.) | |
| 10 mg/ml ADN de sperme de saumon (Q-Biogene) | |

(suite)

| | |
|---|---|
| ***Tampon de pré-hybridation :*** | ***Tampon de lavage 2 :*** |
| 6X SSC | 2X SSC |
| 10X Denhardt's | 0,1 % SDS |
| 10 % Sulfate de Dextran | |
| 0,5 % SDS | |
| $H_2O$ | |
| ***Tampon d'hybridation:*** | ***Tampon de lavage 3 :*** |
| 5X SSC | 0,5X SSC |
| 5X Denhardt's | 0,1 % SDS |
| 0.1 % SDS | |
| $H_2O$ | |
| | ***Tampon de lavage 4 :*** |
| | 1X SSC |
| | 0,1 % SDS |

***- Pré-Hybridation :***

Aliquoter 5ml de tampon de pré-hybridation dans les tubes d'hybridation, ajouter le volume correspondant d'ADN de sperme de saumon pour une concentration finale de 100µg/ml, imbiber les membranes de 5X SSC, placer la membrane dans le tube d'hybridation et pré-hybrider 2 heures à 65°C.

***- Hybridation :***

Eliminer le tampon de pré-hybridation et rincer avec 10-20ml de 5X SSC, éliminer le 5 X SSC, remplacer par 5ml de tampon + ADN de sperme de saumon, dénaturer les sondes RT par 5 mn à 95°C, puis placer sur la glace /1 minute, centrifuger pour reconstitution, puis récupérer le volume approprié de sondes RT dénaturées dans le tube (100.000 à 200.000 cpm/ml), incuber une nuit à 55°C.

***- Lavage :***

Rincer les membranes avec 10-20 ml de tampon de lavage 1, éliminer le tampon et le remplacer par 50 ml de tampon de lavage 2, incuber 30 mn à 55°C, puis éliminer et remplacer en lavant avec le tampon 4, incuber 30mn à 55°C, puis décanter le lavage final, ôter les membranes des tubes, placer sur une cassette et laissez l'acquisition se faire pendant 3 heures.

***4.1.4.5 Acquisition et Analyse d'image***

Les membranes sont placées sur un écran (FX Imaging ScreenK - Bio-rad) pendant 3 heures. Le film est ensuite lu en utilisant un Personal Molecular Imager FX (Bio-rad). L'analyse d'image est réalisée en employant le Safe-Hit Reader Software (COSE).

***4.1.4.6 Calcul de l'Index de Toxicité***

Toutes les données sont transférées dans un programme de calcul automatique qui normalise les différentes membranes et calcule un Index de Toxicité = somme du nombre de gènes up-régulés et down-régulés par un composé donné à une concentration donnée, comparativement aux résultats des contrôles non traités. Les résultats des deux analyses Safe-Hit sont alors comparés et combinés pour évaluer la toxicité potentielle des divers composés testés. Deux paramètres modifiables par l'utilisateur sont impliqués dans le calcul d'Index de Toxicité:

¤ *le Background Threshold (BT)* lisse les signaux faibles, proches du bruit de fond et non attribuables à une expression significative de gène. Il détermine donc le seuil de détection ;
¤ *le Facteur d'Induction (IF)* est déterminé comme étant le facteur multiplicateur, versus les échantillons de contrôle, pour que les clones soient up ou down régulés. La valeur de ce paramètre est généralement 2 ou au dessous de 2 pour obtenir des résultats relevants. L'augmentation progressive de la valeur de l'IF sélectionne les clones qui

sont de plus en plus fortement up ou down régulés.

Le procédé de calcul de l'Index de Toxicité a été développé par comparaison des profils de référence (R : cellules non traitées) avec un profil expérimental (E) et suit les étapes suivantes (voir figure 5) pour une vue schématique du procédé) :

- transformation de toutes les valeurs obtenues en valeurs log,
- calcul de la moyenne des valeurs log pour chacun des essais dupliqués ($M_{iR}$ et $M_{iE}$),
- établissement d'une matrice avec $M_{iR}$ -$M_{iE}$ pour tous les signaux (= $D_i$),
- normalisation des $M_{iE}$ individuelles en soustrayant à $M_{iE}$ la médiane des 14 valeurs proximales de Di (= $NM_{iE}$),
- comparaison des valeurs normalisées avec les valeurs référence ($C_i$ = $NM_{iE}$ - $M_{IR}$),
- transformation exponentielle de $C_i$ (= $F_i$),
- comparaison de $F_i$ avec le Facteur d'Induction choisi par l'utilisateur :

  ¤ si $F_i$ >IF, le gène est considéré comme up-régulé,
  ¤ si 1/IF < $F_i$ < IF, le gène est exprimé sans modification,
  ¤ si $F_i$ < 1/IF, le gène est considéré comme down-régulé.

**4.2. RESULTATS DU TEST AU MTT**

Ces tests ont été réalisés en triplicate sur des hépatocytes primaires de rat exposés pendant 16 heures.

La clomipramine, référencée C218, présente une toxicité importante à 100 μM puisqu'aucune viabilité n'est observée après exposition des cellules pendant 16 heures. En revanche, aucune toxicité n'est observée à 25 μM. A 50 μM, la viabilité supérieure à 80% est tout à fait compatible avec une étude pharmaco-toxicogénomique. Les composés F2695 et F2696 ne présentent aucune cytotoxicité dans ce test, même à la concentration de 100 μM.

Pour réaliser les évaluations pharmaco-toxicogénomiques , 3 concentrations d'un même composé sont utilisées : la concentration qui permet d'obtenir 80% de viabilité (C), ainsi que les concentrations qui correspondent à (C)x10 et à (C)/10.

De façon à comparer la capacité de F2695 et F2696 à produire un score dans le test mis en oeuvre, les mêmes concentrations ont été utilisées pour chacun d'entre eux : 1 μM, 10 μM et 100 μM. Dans le cas de la clomipramine, les concentrations de 1 μM, 10 μM et 50 μM ont été utilisées. Voir les figures 6a, 6b et 6c.

**4.3 RESULTATS SUR HEPATOCYTES PRIMAIRES DE RAT**

Les Index de toxicité (IT) ont été déterminés comme décrit ci-dessus. Dans ces index, n'ont été pris en compte que les clones qui ont été trouvés modulés par rapport aux contrôles dans les deux expériences indépendantes, ne considérant que les clones dont le signal est 2 fois supérieur au bruit de fond (BT). Deux analyses différentes ont été réalisées en prenant deux niveaux de différence (Facteur d'Induction - IF) par rapport à la situation non traitée :

- au moins 1,7 fois par rapport à la situation non traitée. Ce facteur de 1,7 fois représente la valeur la plus faible qui permet de ne pas obtenir d'index lors de la comparaison de deux situations non traitées.
- au moins 2 fois par rapport à la situation non traitée. Ce facteur de 2 fois permet de prendre en compte les signaux les plus robustes.

**4.3.1. Facteur d'induction de 1,7 par rapport à la situation non traitée (tableau 6)**

Tableau 6: Clones « up- et down-régulés » avec des hépatocytes primaires de rat (Facteur d'Induction = 1,7 fois)

| | | | F2695 -1 µM | F2695 10 µM | F2695- 100 µM | F2696 -1 µM | F2696 10µM | F2696 100µ M | C 218- 1µM | C218- 10µM | C 218- 100µM | |
|---|---|---|---|---|---|---|---|---|---|---|---|---|
| Up | >1,7 | Up | | | 1 | | | 15 | 2 | 2 | 13 | |
| Down | <0,58 8 | Down | | | 1 | 2 | 5 | 7 | 7 | 13 | 15 | |
| | | TI | | | 2 | 2 | 5 | 22 | 9 | 15 | 28 | |
| | | | | | | | | | | | | |
| Pos | nb U | nb D | | | | | | | | | | Gene |
| A09 | **3** | | | | | | | | 2,90 | 2,23 | 2,14 | H. sapiens mitochondrion, 12S |
| A20 | | 1 | | | | | | 0,56 | | | | H. sapiens initiation factor eIF-5A gene |
| B20 | | 2 | | | | | | | | 0,14 | 0,27 | H. sapiens chromosome 19., BAC CIT-B-191n6 |
| B22 | | 2 | | | | | | | | 0,17 | 0,32 | H. sapiens Genomic sequence from 17 |
| C01 | **4** | | | | | | | 3,20 | 1,93 | 1,82 | 1,91 | H. sapiens mitochondrion, 16S |
| E01 | **1** | | | | | | | | | | 1,73 | H. sapiens mRNA for lipocortin II |
| E05 | | 2 | | | | | | | | 0,22 | 0,35 | H. sapiens DNA sequence from clone 740A11 on chromosome Xq22.2-23. Contains part of the |

(suite)

| | | | F2695 -1 µM | F2695 10 µM | F2695- 100 µM | F2696 -1 µM | F2696 10µM | F2696 100µ M | C 218- 1µM | C218- 10µM | C 218- 100µM | |
|---|---|---|---|---|---|---|---|---|---|---|---|---|
| Up | >1,7 | Up | | | 1 | | | 15 | 2 | 2 | 13 | |
| Down | <0,58 8 | Down | | | 1 | 2 | 5 | 7 | 7 | 13 | 15 | |
| | | TI | | | 2 | 2 | 5 | 22 | 9 | 15 | 28 | |
| | | | | | | | | | | | | |
| | | | | | | | | | | | | COL4A5 gene for Collagen Alpha 5 (IV) Chain Precursor. Contains GSS1, complete sequence |
| E11 | **1** | | | | | | | 2,12 | | | | H. sapiens chlordecone reductase homolog liver, mRNA |
| E19 | **1** | | | | | | | | | | 1,72 | H. sapiens mitochondrion, cytochrome c oxidase subunit 1 |
| E21 | | 2 | | | | | | | | 0,5.6 | 0,58 | H. sapiens ribosomal protein S14 gene |
| F24 | **1** | 1 | | | | | | | | | 0,52 | H. sapiens LIM homeobox protein cofactor (CLIM-1) mRNA |

(suite)

| | | | F2695 -1 µM | F2695 10 µM | F2695-100 µM | F2696 -1 µM | F2696 10µM | F2696 100µ M | C 218- 1µM | C218-10µM | C 218-100µM | |
|---|---|---|---|---|---|---|---|---|---|---|---|---|
| Up | >1,7 | Up | | | 1 | | | 15 | 2 | 2 | 13 | |
| Down | <0,58 8 | Down | | | 1 | 2 | 5 | 7 | 7 | 13 | 15 | |
| | | TI | | | 2 | 2 | 5 | 22 | 9 | 15 | 28 | |
| | | | | | | | | | | | | |
| G01 | | | | | | | | | | | 2,04 | H. sapiens estrogen receptor-related protein (variant ER from breast cancer) mRNA |
| G05 | **1** | | | | | | | | | | 2,02 | H. sapiens mitochondrion, cytochrome c oxidase subunit 1 |
| G09 | **2** | | | | | | | 2,09 | | | 1,76 | H. sapiens mitochondrion, cytochrome b |
| I01 | **1** | | | | | | | 2,05 | | | | H. sapiens mitochondrion, cytochrome c oxidase subunit 1 |
| I18 | **2** | | | | | | | 2,38 | | | 1,88 | H. sapiens 18S rRNA gene |
| L01 | **1** | | | | | | | 2,05 | | | | H. sapiens divalent cation tolerant protein CUTA mRNA |
| L22 | **1** | | | | | | | 1,78 | | | | H. sapiens mRNA for Lon protease-like protein |

(suite)

| | | | F2695 -1 µM | F2695 10 µM | F2695-100 µM | F2696 -1 µM | F2696 10µM | F2696 100µ M | C 218- 1µM | C218-10µM | C 218-100µM | |
|---|---|---|---|---|---|---|---|---|---|---|---|---|
| Up | >1,7 | Up | | | 1 | | | 15 | 2 | 2 | 13 | |
| Down | <0,58 8 | Down | | | 1 | 2 | 5 | 7 | 7 | 13 | 15 | |
| | | TI | | | 2 | 2 | 5 | 22 | 9 | 15 | 28 | |
| | | | | | | | | | | | | |
| L23 | **1** | | | | | | | | | | 1,75 | H. sapiens cDNA NIH_MGC_16 clone IMAGE: 3350241 5', mRNA sequence |
| M07 | **2** | | | | | | | 2,25 | | | 1,75 | H. sapiens mitochondrion, cytochrome c oxidase subunit 1 |
| M12 | | 3 | | | | | | 0,21 | | 0,16 | 0,39 | H. sapiens mRNA; cDNA DKFZp564C1563 |
| M23 | **1** | | | | | | | 1,95 | | | | Sequence 21 from patent US 5851764 |
| P05 | **1** | | | | | | | 1,78 | | | | H. sapiens PAC clone DJ404K21 from Xq23 |
| Q11 | **2** | | | | 1,81 | | | | | | 1,92 | unk |
| Q24 | **1** | | | | | | | 1,77 | | | | H. sapiens 28S ribosomal RNA gene |
| S01 | **1** | | | | | | | | | | 2,98 | Mus musculus TCR beta locus |

(suite)

| | | | F2695 -1 µM | F2695 10 µM | F2695- 100 µM | F2696 -1 µM | F2696 10µM | F2696 100µ M | C 218- 1µM | C218- 10µM | C 218- 100µM | |
|---|---|---|---|---|---|---|---|---|---|---|---|---|
| Up | >1,7 | Up | | | 1 | | | 15 | 2 | 2 | 13 | |
| Down | <0,58 8 | Down | | | 1 | 2 | 5 | 7 | 7 | 13 | 15 | |
| | | TI | | | 2 | 2 | 5 | 22 | 9 | 15 | 28 | |
| | | | | | | | | | | | | |
| T08 | | 6 | | | | 0,50 | 0,22 | 0,20 | 0,35 | 0,14 | 0,22 | H. sapiens mRNA for KIAA 1185 protein |
| U04 | | 6 | | | | 0,57 | 0,26 | 0,19 | 0,48 | 0,22 | 0,37 | H. sapiens translation initiation factor eIF-2alpha mRNA |
| V22 | | | | | | | | | | | | H. sapiens mRNA for elongation, factor 1-alpha (clone CEF4) |
| W17 | **1** | | | | | | | 2,96 | | | | H. sapiens mitochondrion, hypoxia inducible gene-14 |
| X02 | | 5 | | | | | 0,29 | 0,20 | 0,36 | 0,24 | 0,31 | unk |
| X05 | | 2 | | | | | | | | 0,15 | 0,24 | H. sapiens microsomal epoxide hydrolase (EPHX) gene |
| X06 | **1** | 5 | | | | | 0,2 | 0,16 | 0,23 | 0,15 | 0,23 | H. sapiens Genomic séquence from 9q34 |
| X23 | **1** | | | | | | | 1,92 | | | | unk |

(suite)

| | | | F2695 -1 µM | F2695 10 µM | F2695- 100 µM | F2696 -1 µM | F2696 10µM | F2696 100µ M | C 218- 1µM | C218- 10µM | C 218- 100µM | |
|---|---|---|---|---|---|---|---|---|---|---|---|---|
| Up | >1,7 | Up | | | 1 | | | 15 | 2 | 2 | 13 | |
| Down | <0,58 8 | Down | | | 1 | 2 | 5 | 7 | 7 | 13 | 15 | |
| | | TI | | | 2 | 2 | 5 | 22 | 9 | 15 | 28 | |
| | | | | | | | | | | | | |
| Y17 | **1** | | | | | | | 2,65 | | | | H. sapiens 28S ribosomal RNA gene |
| Z13 | | 3 | | | | | | | 0,34 | 0,29 | 0,27 | unk |
| Z20 | | 1 | | | | | | | | | 0,57 | Homo sapiens cDNA wc44h09,x1 NCI_ CGAP-Pr28 clone IMAGE:2321537 3' similar to SW:RB24_Mouse P35290 RAS_ RELATED PROTEIN RAB-24;, mRNA sequence |
| AA11 | | 3 | | | | | | | 0,38 | 0,27 | 0,31 | H. sapiens Repeat sequence AluJb fragment inserted into a cDNA coding for an unknown protein |
| AA13 | **1** | | | | | | | | | | 1,79 | H. sapiens 18S rRNA gene, |
| AC13 | | 5 | | | | | 0,22 | 0,16 | 0,28 | 0,16 | 0,28 | H. sapiens 7S RNA L gene |

Les index de toxicité suivants ont été obtenus :

| F2695 | Index de Toxicité |
|---|---|
| 1 μM | 0 |
| 10 μM | 0 |
| 100 μM | 17 |

| F2696 | Index de Toxicité |
|---|---|
| 1 μM | 2 |
| 10 μM | 5 |
| 100 μM | 22 |

| C218 | Index de Toxicité |
|---|---|
| 1μM | 9 |
| 10 μM | 15 |
| 50 μM | 28 |

Le classement suivant peut donc être établi, du plus toxique au moins toxique C218 (clomipramine) > F2696 >>> F2695.

La clomipramine, molécule de référence codée dans le présent essai C218, montre une augmentation des signatures en relation avec les concentrations testées : respectivement 9, 15 et 28 signatures aux concentrations de 1, 10 et 50 μM (concentration maximale définie dans le test préalable de cytotoxicité). Fort logiquement, la totalité des signatures survenant aux concentrations faible et moyenne est retrouvée aux concentrations supérieures.

Aux concentrations de 1 et 10 μM, le F2695 n'induit aucune signature sur les 682 possibles signatures de stress testées dans le présent essai. A la concentration la plus élevée de 100 μM, seules sont identifiées deux signatures, dont une commune avec le C218 mais de signification inconnue.

Le F2696 montre une augmentation des signatures en relation avec les concentrations testées : respectivement 2, 5 et 22 signatures aux concentrations de 1, 10 et 100 μM. La totalité des signatures survenant aux concentrations faible et moyenne est retrouvée aux concentrations supérieures. Aucune des 22 signatures n'est commune avec le F2695. En revanche, les signatures qui apparaissent aux concentrations faible et moyenne (5 dont les 2 présentes dès la faible concentration), font toutes les 5 partie des 9 signatures objectivées pour la clomipramine dès la faible dose de 1 μM. A la forte concentration de 100 μM, 10/26 signatures du F2696 sont retrouvées parmi les 28 identifiées avec la clomipramine à 50 μM.

Sur le plan qualitatif, est à souligner pour le F2696 et la clomipramine l'impact sur les transcrits mitochondriaux, notamment au niveau de la Cox1 et du cytochrome b. Ces signatures ne sont pas présentes avec le F2695 (positions G05/G09/I01).

### 4.3.2. Facteur d'induction de 2 par rapport à la situation non traitée (tableau 7)

Tableau 7: Clones « up- et down-régulés » avec des hépatocytes primaires de rat (Facteur d'Induction = 2 fois)

| | | | F2695- 1 µM | F2695- 10µM | F2695- 100µM | F2696- 1 µM | F2696- 10µM | F2696- 100µM | C218- 1µM | C 218- 10µM | C218- 100 µM | |
|---|---|---|---|---|---|---|---|---|---|---|---|---|
| Up | >1,7 | Up | | | | | | 10 | 1 | 1 | 4 | |
| Down | <0,588 | Down | | | | | 5 | 6 | 7 | 12 | 12 | |
| | | TI | | | | | 5 | 16 | 8 | 13 | 16 | |
| | | | | | | | | | | | | |
| Pos | nb U | nb D | | | | | | | | | | Gene |
| A09 | **3** | | | | | | | | 2,90 | 2,23 | 2,14 | H. sapiens mitochondrion, 12S |
| B20 | | **2** | | | | | | | | 0,14 | 0,27 | H. sapiens chromosome 19, BAC CIT-B-191n6 |
| B22 | | **2** | | | | | | | | 0,17 | 0,32 | H. sapiens Genomic sequence from 17 |
| C01 | **1** | | | | | | | 3,20 | | | | H. sapiens mitochondrion, 16S |
| E05 | | **2** | | | | | | | | 0,22 | 0,35 | H. sapiens DNA sequence from clone 740A11 on chromosome Xq22.2-23. Contains part of the COL4A5 gene for Collagen Alpha 5 (IV) Chain Precursor. Contains GSS1, complet sequence |

(suite)

| | | | F2695- 1 µM | F2695- 10µM | F2695- 100µM | F2696- 1 µM | F2696- 10µM | F2696- 100µM | C218- 1µM | C 218- 10µM | C218- 100 µM | |
|---|---|---|---|---|---|---|---|---|---|---|---|---|
| Up | >1,7 | Up | | | | | | 10 | 1 | 1 | 4 | |
| E11 | **1** | | | | | | | 2,12 | | | 2,04 | H. sapiens chlordecone reductase homolog liver, mRNA |
| G01 | **1** | | | | | | | | | | 2,04 | H. sapiens estrogen receptor-related protein (variant ER from breast cancer) MRNA |
| G05 | | | | | | | | | | | 2,02 | H. sapiens subunit 1 |
| G09 | **1** | | | | | | | 2,09 | | | | mitochondrion, cytochrome c oxidase H. sapiens mitochondrion, cytochrome b |
| I01 | **1** | | | | | | | 2,05 | | | | H. sapiens mitochondrion, cytochrome c oxidase subunit 1 |
| I18 | **1** | | | | | | | 2,38 | | | | H. sapiens 18S rRNA gene |
| J03 | **1** | | | | | | | 2,12 | | | | H. sapiens CLP mRNA |
| L01 | **1** | | | | | | | 2,05 | | | | H. sapiens divalent cation tolerant protein CUTA mRNA |

(suite)

| | | | F2695- 1 µM | F2695- 10µM | F2695- 100µM | F2696- 1 µM | F2696- 10µM | F2696- 100µM | C218- 1µM | C 218- 10µM | C218- 100 µM | |
|---|---|---|---|---|---|---|---|---|---|---|---|---|
| Up | >1,7 | Up | | | | | | 10 | 1 | 1 | 4 | |
| M07 | **1** | | | | | | | 2,25 | | | | H. sapiens mitochondrion, cytochrome c oxidase subunit 1 |
| M12 | | **3** | | | | | | 0,21 | | 0,16 | 0,39 | H. sapiens mRNA; cDNA DKFZp564C1563 |
| S01 | **1** | | | | | | | | | | 2,98 | Mus muculus TCR beta locus |
| T08 | | **5** | | | | | 0,22 | 0,20 | 0,35 | 0,14 | 0,22 | H. sapiens mRNA for KIAA1185 protein |
| U04 | | **5** | | | | | 0,26 | 0,19 | 0,48 | 0,22 | 0,37 | H. sapiens translation initiation factor eIF-2alpha mRNA |
| W17 | **1** | | | | | | | 2,96 | | | | H. sapiens mitochondrion, hypoxia inducible gene-14 |
| X02 | | **5** | | | | | 0,29 | 0,20 | 0,36 | 0,24 | 0,31 | unk |
| X05 | | **2** | | | | | | | | 0,15 | 0,24 | H. sapiens microsomal epoxide hydrolase (EPHX) gene |
| X06 | | **5** | | | | | 0,20 | 0,16 | 0,23 | 0,15 | 0,23 | H. sapiens Genomic sequence from 9q34 |

(suite)

| | | | F2695- 1 µM | F2695- 10µM | F2695- 100µM | F2696- 1 µM | F2696- 10µM | F2696- 100µM | C218- 1µM | C 218- 10µM | C218- 100 µM | |
|---|---|---|---|---|---|---|---|---|---|---|---|---|
| Up | >1,7 | Up | | | | | | 10 | 1 | 1 | 4 | |
| Y17 | **1** | | | | | | | 2,65 | | | | H. sapiens 28S ribosomal RNA gene |
| Z13 | | **3** | | | | | | | 0,34 | 0,29 | 0,27 | unk |
| AA11 | | **3** | | | | | | | 0,38 | 0,27 | 0,31 | H. sapiens Repeat sequence AluJb fragment inserted into a cDNA coding for an unknown protein |
| AC13 | | **5** | | | | | 0,22 | 0,16 | 0,28 | 0,16 | 0,28 | H. sapiens 7S RNA L gene |

Les index suivants ont été obtenus :

| F2695 | Index de Toxicité |
|---|---|
| 1µM | 0 |
| 10 µM | 0 |
| 100 µM | 0 |

| C218 | Index de Toxicité |
|---|---|
| 1µM | 8 |
| 10µM | 13 |
| 30µM | 16 |

| F2696 | Index de Toxicité |
|---|---|
| 1 µM | 0 |
| 10µM | 5 |
| 100 µM | 16 |

Selon ces paramètres, le classement suivant peut être proposé, du plus toxique au moins toxique : C218 (clomipramine) > F2696 >>>>> F2695.

En considérant les clones sur- ou sous-exprimés d'un facteur 2, le F2695 n'induit aucune signature, même à la concentration de 100 µM.

L'effet de la concentration sur l'apparition des signatures se confirme par la disparition des signatures de faible intensité pour le F2696 à 1 µM, présentes dans l'analyse précédente avec un facteur d'induction de 1,7.

Sur le plan qualitatif, l'impact du F2696 et de la clomipramine sur la Cox1 et le cytochrome b est également confirmé (positions G05/G09/I01).

Le F2695, énantiomère pharmacologiquement actif du F2207, est dépourvu d'impact significatif dans ce test, alors que la clomipramine est utilisée comme produit de référence témoin positif.

En revanche, le F2696, énantiomère inactif du F2207 présente un profil de signatures quantitativement et qualitativement proche de la clomipramine, et ne présente aucune signature commune avec le F2695.

Tout ceci témoigne d'un meilleur profil toxicogénomique pour l'énantiomère actif F2695 dont, sur ce modèle expérimental, le coefficient de sécurité est très significativement meilleur que pour le F2696.

## 4.4 CONCLUSION

Les études pharmaco-toxicogénomiques réalisées sur les molécules F2695 et F2696, énantiomères du F2207 (aux concentrations de 10, 50 et 100 µM), et C218 (clomipramine, aux concentrations de 1, 10 et 50 µM), à partir d'hépatocytes de rat en primoculture, ont permis d'obtenir des signatures de stress et des index de toxicité concentration-dépendants. Ces études confirment la capacité du test pharmaco-toxicogénomique à révéler des signatures de stress dans des conditions de traitement (concentrations, durée de traitement) qui ne provoquent aucune toxicité dans un test classique de viabilité tel que le MTT.

Plusieurs faits marquants se dégagent de cette étude:

¤ sur ce modèle d'hépatocytes primaires de rat, seul le F2695, énantiomère pharmacologiquement actif du F2207, n'induit pas d'index de toxicité significatif;

¤ le F2696, énantiomère inactif du F2207, et la clomipramine, psychotrope de référence, induisent des index importants formés de signatures de stress communes ou très proches. Dans ce système, la clomipramine, molécule de référence produit positif, est le produit induisant le plus de signatures de stress, des index significatifs étant obtenus dès les concentrations les plus faibles. A ce titre, il est intéressant de mentionner que la clomipramine peut induire un certain nombre d'effets indésirables chez l'homme tels par exemple la tachycardie, l'hypotension orthostatique, des troubles de la conduction ou du rythme, et exceptionnellement des hépatites. En cas de surdosage accidentel à la clomipramine, on peut observer entre autres des syncopes, des troubles hématologiques, des manifestations cardiovasculaires sévères.

Sans préjuger d'une identité de mécanisme physiopathologique, il est intéressant de noter que F2696 présente des signatures de stress communes ou très proches de celles de la clomipramine et induit également des effets indésirables tels que les troubles cardiovasculaires précédemment décrits.

Il est ainsi légitime d'avancer que les signatures observées sont indépendantes de tout profil antidépresseur ou plus largement psychotrope. Elles doivent bien en revanche être considérées comme des « signatures de stress » (le F2696 provoque en particulier une diminution d'expression d'un gène impliqué dans la synthèse protéique et d'un facteur d'initiation de translation). Tout ceci témoigne d'un meilleur profil toxicogénomique pour l'énantiomère actif F2695 dont, sur ce modèle expérimental, le coefficient de sécurité est très significativement meilleur que pour le F2696.

## Revendications

1. L'énantiomère (1S,2R) du Milnacipran (Z(±)-2-(amino méthyl)-N,N-diéthyl-1-phénylcyclopropanecarboxamide), ou un de ses sels pharmaceutiquement acceptables, pour utilisation comme médicament destiné à prévenir ou traiter la dépression, les états dépressifs, la fibromyalgie, le syndrome de fatigue chronique, la douleur, chez des patients présentant des antécédents cardiovasculaires et/ou atteints de troubles cardiovasculaires, administré à une dose comprise entre 0.01 mg et 10mg/kg de poids corporel par jour en une ou plusieurs prises.

2. Enantiomère (1S,2R) du Milnacipran (Z(±)-2-(amino méthyl)-N,N-diéthyl-1-phénylcyclopropanecarboxamide), ou un de ses sels pharmaceutiquement acceptables, selon la revendication 1, **caractérisé en ce que** la dose administrée est comprise entre 0.05 mg et 5 mg/ kg de poids corporel par jour en une ou plusieurs prises.

3. Enantiomère (1S,2R) du Milnacipran (Z(±)-2-(amino méthyl)-N,N-diéthyl-1-phénylcyclopropanecarboxamide), ou un de ses sels pharmaceutiquement acceptables selon la revendication 1 ou 2 **caractérisé en ce que** la dose administrée est comprise entre 0.1mg et 1 mg/ kg de poids corporel par jour en une ou plusieurs prises.

4. Enantiomère (1S,2R) du Milnacipran (Z(±)-2-(amino méthyl)-N,N-diéthyl-1-phénylcyclopropanecarboxamide), ou un de ses sels pharmaceutiquement acceptables selon l'une quelconque des revendications 1 à 3, **caractérisé en ce que** les troubles cardiovasculaires correspondent à une élévation de la pression artérielle et/ou une augmentation de la fréquence cardiaque.

5. Enantiomère (1S,2R) du Milnacipran (Z(±)-2-(amino méthyl)-N,N-diéthyl-1-phénylcyclopropanecarboxamide), ou un de ses sels pharmaceutiquement acceptables selon la revendication 4, **caractérisé en ce que** l'élévation de la pression artérielle correspond à une élévation de la pression artérielle diastolique.

6. Enantiomère (1S,2R) du Milnacipran (Z(±)-2-(amino méthyl)-N,N-diéthyl-1-phénylcyclopropanecarboxamide), ou un de ses sels pharmaceutiquement acceptables selon l'une quelconque des revendications 1 à 5, tout en limitant en outre les risques de toxicité organique et/ou tissulaire.

7. Enantiomère (1S,2R) du Milnacipran (Z(±)-2-(amino méthyl)-N,N-diéthyl-1-phénylcyclopropanecarboxamide), ou un de ses sels pharmaceutiquement acceptables selon l'une quelconque des revendications 1 à 6 **caractérisé en ce que** le dit énantiomère (1S,2R) du Milnacipran est le chlorhydrate de Z-(1S,2R)-2-(aminométhyl)-N,N-diéthyl-1-phénylcyclopropanecarboxamide (F2695).

8. Enantiomère (1S,2R) du Milnacipran (Z(±)-2-(amino métlyl)-N,N-diéthyl-1-phenylcyclopropanecarboxamide), ou un de ses sels pharmaceutiquement acceptables selon l'une quelconque des revendications 1 à 7, pour le traitement ou la prévention de la dépression profonde, la dépression résistante, la dépression du sujet âgé, la dépression psychotique, la dépression induite par des traitements interféron, l'état dépressif, le syndrome maniaco-dépressif, les épisodes dépressifs saisonniers, les épisodes dépressifs liés à une condition médicale générale ou les épisodes dépressifs liés à des substances agissant sur l'humeur.

9. Enantiomère (1S,2R) du Milnacipran (Z(±)-2-(amino méthyl)-N,N-diéthyl-1-phénylcyclopropanecarboxamide), ou un de ses sels pharmaceutiquement acceptables selon l'une quelconque des revendications 1 à 7, pour le traitement ou la prévention de la douleur chronique.

**10.** Enantiomère (1S,2R) du Milnacipran (Z(±)-2-(amino méthyl)-N,N-diéthyl-1-phénylcyclopropanecarboxamide), ou un de ses sels pharmaceutiquement acceptables selon l'une quelconque des revendications 1 à 9, **caractérisé en ce que** les antécédents cardiovasculaires et/ou troubles cardiovasculaires sont choisis parmi l'infarctus du myocarde, les troubles du rythme cardiaque (tachycardie, bradycardie, palpitations), les troubles de la pression artérielle (patients hypo- ou hypertendus) et les cardiopathies.

**11.** Enantiomère (1S,2R) du Milnacipran (Z(±)-2-(amino méthyl)-N,N-diéthyl-1-phéhylcyclopropanecarboxamide), ou un de ses sels pharmaceutiquement acceptables selon l'une quelconque des revendications 1 à 10, **caractérisé en ce que** le médicament contient:

a) ledit énantiomère (1S,2R) du Milnacipran ou un de ses sels pharmaceutiquement acceptables, et
b) au moins un composé actif choisi parmi les psychotropes, notamment les anti-dépresseurs, et les agents anti-muscariniques,

comme produits de combinaison pour une utilisation simultanée, séparée ou échelonnée dans le temps pour le traitement ou la prévention de la dépression, notamment la dépression profonde, la dépression résistante, la dépression du sujet âgé, la dépression psychotique, la dépression induite par des traitements interféron, l'état dépressif, le syndrome maniaco-dépressif, les épisodes dépressifs saisonniers, les épisodes dépressifs liés à une condition médicale générale, les épisodes dépressifs liés à des substances agissant sur l'humeur.

## Claims

**1.** The (1S,2R) enantiomer of milnacipran (Z(±)-2-(aminomethyl)-N,N-diethyl-1-phenylcyclopropane-carboxamide) or a pharmaceutically acceptable salt thereof, for use as a medicament intended for preventing or treating depression, depressive states, fibromyalgia, chronic fatigue syndrome, pain, in patients having a history of cardiovascular disease and/or suffering from cardiovascular disorders, administered at a dose of between 0.01 mg and 10 mg/kg of body weight per day in one or more intakes.

**2.** (1S,2R) enantiomer of milnacipran (Z(±)-2-(aminomethyl)-N,N-diethyl-1-phenylcyclopropane-carboxamide) or a pharmaceutically acceptable salt thereof, according to Claim 1, **characterized in that** the dose administered is between 0.05 mg and 5 mg/kg of body weight per day in one or more intakes.

**3.** (1S,2R) enantiomer of milnacipran (Z(±)-2-(aminomethyl)-N,N-diethyl-1-phenylcyclopropane-carboxamide) or a pharmaceutically acceptable salt thereof, according to Claim 1 or 2, **characterized in that** the dose administered is comprised between 0.1 mg and 1 mg/kg of body weight per day in one or more doses.

**4.** (1S,2R) enantiomer of milnacipran (Z(±)-2-(aminomethyl)-N,N-diethyl-1-phenylcyclopropane-carboxamide) or a pharmaceutically acceptable salt thereof, according to any one of Claims 1 to 3, **characterized in that** the cardiovascular disorders correspond to an increase in arterial blood pressure and/or an increase in heart rate.

**5.** (1S,2R) enantiomer of milnacipran (Z(±)-2-(aminomethyl)-N,N-diethyl-1-phenylcyclopropane-carboxamide) or a pharmaceutically acceptable salt thereof, according to Claim 4, **characterized in that** the increase in arterial blood pressure corresponds to an increase in diastolic arterial blood pressure.

**6.** (1S,2R) enantiomer of milnacipran (Z(±)-2-(aminomethyl)-N,N-diethyl-1-phenylcyclopropane-carboxamide) or a pharmaceutically acceptable salt thereof, according to any one of Claims 1 to 5, while limiting moreover the risk of organ and/or tissue toxicity.

**7.** (1S,2R) enantiomer of milnacipran (Z(±)-2-(aminomethyl)-N,N-diethyl-1-phenylcyclopropane-carboxamide) or a pharmaceutically acceptable salt thereof, according to any one of Claims 1 to 6, **characterized in that** said (1S, 2R) enantiomer of milnacipran is the hydrochloride of Z-(1S,2R)-2-(aminomethyl)-N,N-diethyl-1-phenylcyclopropane-carboxamide (F2695).

**8.** (1S,2R) enantiomer of milnacipran (Z(±)-2-(aminomethyl)-N,N-diethyl-1-phenylcyclopropane-carboxamide) or a pharmaceutically acceptable salt thereof, according to any one of Claims 1 to 7, for the treatment or prevention of deep depression, resistant depression, depression in the elderly, psychotic depression, depression induced by treatments with interferon, depressive state, manic-depressive syndrome, seasonal depressive episodes, depressive

episodes related to general health status, or depressive episodes related to mood-altering substances.

**9.** (1S,2R) enantiomer of milnacipran (Z(±)-2-(aminomethyl)-N,N-diethyl-1-phenylcyclopropane-carboxamide) or a pharmaceutically acceptable salt thereof, according to any one of Claims 1 to 7, for the treatment or prevention of chronic pain.

**10.** (1S,2R) enantiomer of milnacipran (Z(±)-2-(aminomethyl)-N,N-diethyl-1-phenylcyclopropane-carboxamide) or a pharmaceutically acceptable salt thereof, according to any one of Claims 1 to 9, **characterized in that** the history of cardiovascular disease and/or cardiovascular disorders is (are) chosen from myocardial infarction, cardiac rhythm disorders (tachycardia, bradycardia, palpitations), arterial blood pressure disorders (hypo- or hypertensive patients) and cardiopathies.

**11.** (1S,2R) enantiomer of milnacipran (Z(±)-2-(aminomethyl)-N,N-diethyl-1-phenylcyclopropane-carboxamide) or a pharmaceutically acceptable salt thereof, according to any one of Claims 1 to 10, **characterized in that** the medicament contains:

a) said (1S,2R) enantiomer of milnacipran or a pharmaceutically acceptable salt thereof, and
b) at least one active compound chosen from psychotropic agents, in particular antidepressants, and antimuscarinic agents,

as combination products for use simultaneously, separately or staggered in time for the treatment or prevention of depression, notably deep depression, resistant depression, depression in the elderly, psychotic depression, depression induced by treatments with interferon, depressive state, manic-depressive syndrome, seasonal depressive episodes, depressive episodes related to general health status, and depressive episodes related to mood-altering substances.

## Patentansprüche

**1.** Das (1S,2R)-Enantiomer von Milnacipran (Z(±)-2-(Aminomethyl)-N,N-diethyl-1-phenytcyclopropancarboxamid) oder eines von dessen pharmazeutisch annehmbaren Salzen zur Verwendung als Arzneimittel, welches dazu bestimmt ist, Depression, depressive Zustände, Fibromyalgie, das chronische Müdigkeitssyndrom, Schmerzen bei Patienten, welche eine Herz-Kreislauf-Vorgeschichte aufweisen und/oder an Herz-Kreislauf-Störungen leiden, zu verhüten oder zu behandeln, wobei dieses in einer Dosis zwischen 0,01 mg und 10 mg/kg Körpergewicht pro Tag eingeschlossen in einer oder mehreren Einnahmen verabreicht wird.

**2.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach Anspruch 1, **dadurch gekennzeichnet, dass** die verabreichte Dosis zwischen 0,05 mg und 5 mg/kg Körpergewicht pro Tag eingeschlossen in einer oder mehreren Einnahmen liegt.

**3.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach Anspruch 1 oder 2, **dadurch gekennzeichnet, dass** die verabreichte Dosis zwischen 0,1 mg und 1 mg/kg Körpergewicht pro Tag eingeschlossen in einer oder mehreren Einnahmen liegt.

**4.** (1 S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach einem der Ansprüche 1 bis 3, **dadurch gekennzeichnet, dass** die Herz-Kreislauf-Störungen einer Erhöhung des arteriellen Drucks und/oder einer Erhöhung der Herzfrequenz entsprechen.

**5.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach Anspruch 4, **dadurch gekennzeichnet, dass** die Erhöhung des arteriellen Drucks einer Erhöhung des diastolischen arteriellen Drucks entspricht.

**6.** (1 S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach einem der Ansprüche 1 bis 5, wobei außerdem die Risiken von Organ- und/oder Gewebetoxizität begrenzt werden.

**7.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach einem der Ansprüche 1 bis 6, **dadurch gekennzeichnet, dass** das (1 S,2R)-Enantiomer von Milnacipran das Chlorhydrat von Z-(1 S,2R)-2-(Aminomethyl)-N,N-diethyl-1-phenylcyclopropancarboxamid (F2695) ist.

**8.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach einem der Ansprüche 1 bis 7 zur Behandlung oder Verhütung von tiefer Depression, resistenter Depression, Altersdepression, psychotischer Depression, der Depression, welche durch Interferon-Behandlungen induziert wird, dem depressiven Zustand, dem manisch-depressiven Syndrom, den saisonalen depressiven Episoden, den depressiven Episoden, die mit einem generellen medizinischen Zustand in Zusammenhang stehen, oder den depressiven Episoden, die mit Substanzen, die auf die Gemütsverfassung wirken, in Zusammenhang stehen.

**9.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach einem der Ansprüche 1 bis 7 zur Behandlung oder Verhütung von chronischem Schmerz.

**10.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach einem der Ansprüche 1 bis 9, **dadurch gekennzeichnet, dass** die Herz-Kreislauf-Vorgeschichte und/oder Herz-Kreislauf-Störungen ausgewählt sind aus Myokardinfarkt, Herzrhythmusstörungen (Tachykardie, Bradykardie, Palpitationen), Störungen des arteriellen Drucks (hypo- oder hypertensive Patienten) und den Kardiopathien.

**11.** (1S,2R)-Enantiomer von Milnacipran (Z(±)-2-(Aminomethyl)-N,N-diethyl-1-phenylcyclopropancarboxamid) oder eines von dessen pharmazeutisch annehmbaren Salzen nach einem der Ansprüche 1 bis 10, **dadurch gekennzeichnet, dass** das Arzneimittel enthält:

a) das (1S,2R)-Enantiomer von Milnacipran oder eines von dessen pharmazeutisch annehmbaren Salzen und
b) wenigstens einen Wirkstoff, ausgewählt unter den Psychopharmaka, insbesondere den Antidepressiva und den antimuskarinischen Mitteln,

als Kombinationsprodukte für eine gleichzeitige, getrennte oder zeitlich gestaffelte Verwendung für die Behandlung oder die Verhütung von Depression, insbesondere tiefer Depression, resistenter Depression, Altersdepression, psychotischer Depression, der Depression, welche durch Interferon-Behandlungen induziert wird, dem depressiven Zustand, dem manisch-depressiven Syndrom, den saisonalen depressiven Episoden, den depressiven Episoden, die mit einem generellen medizinischen Zustand in Zusammenhang stehen, den depressiven Episoden, die mit Substanzen, die auf die Gemütsverfassung wirken, in Zusammenhang stehen.

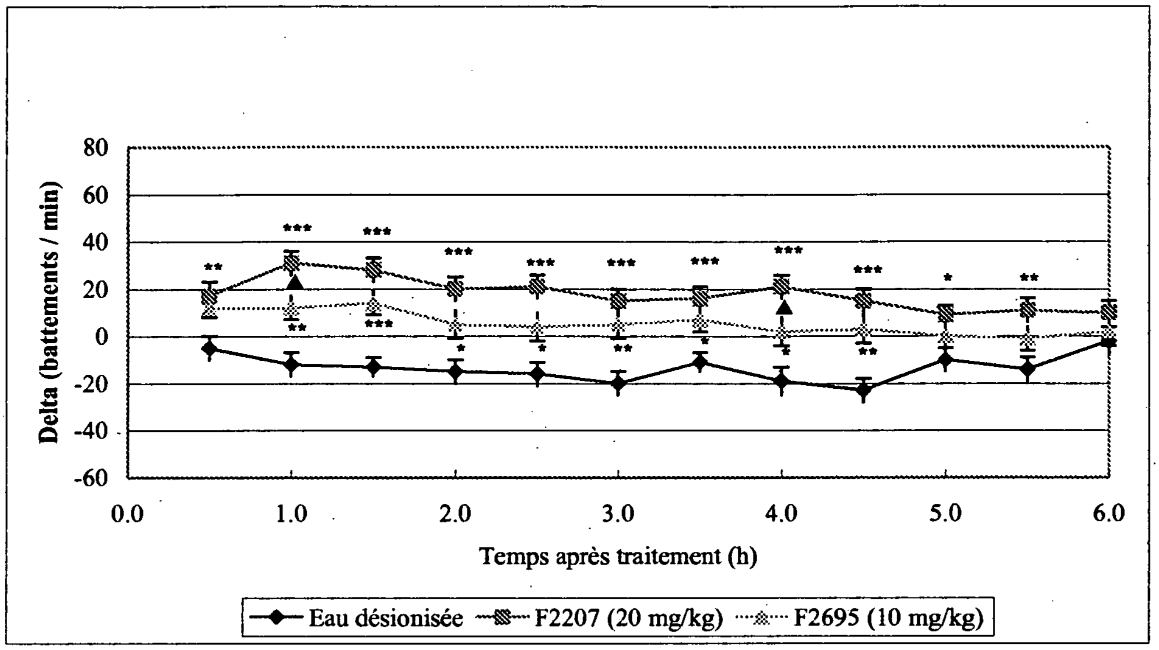


**FIGURE 1**

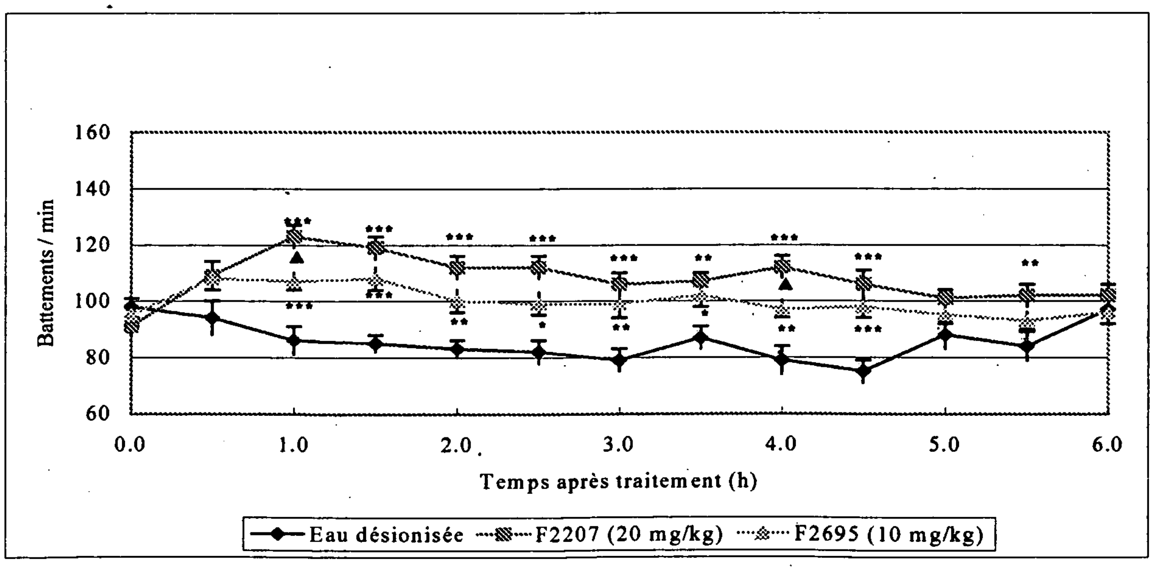


**FIGURE 2**

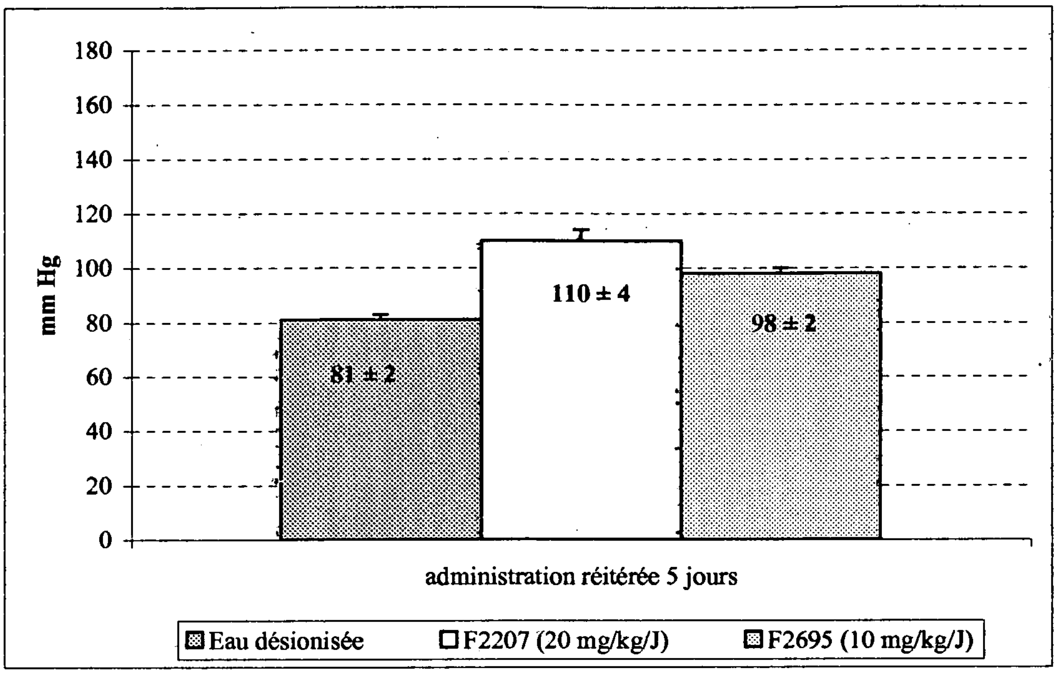


FIGURE 3

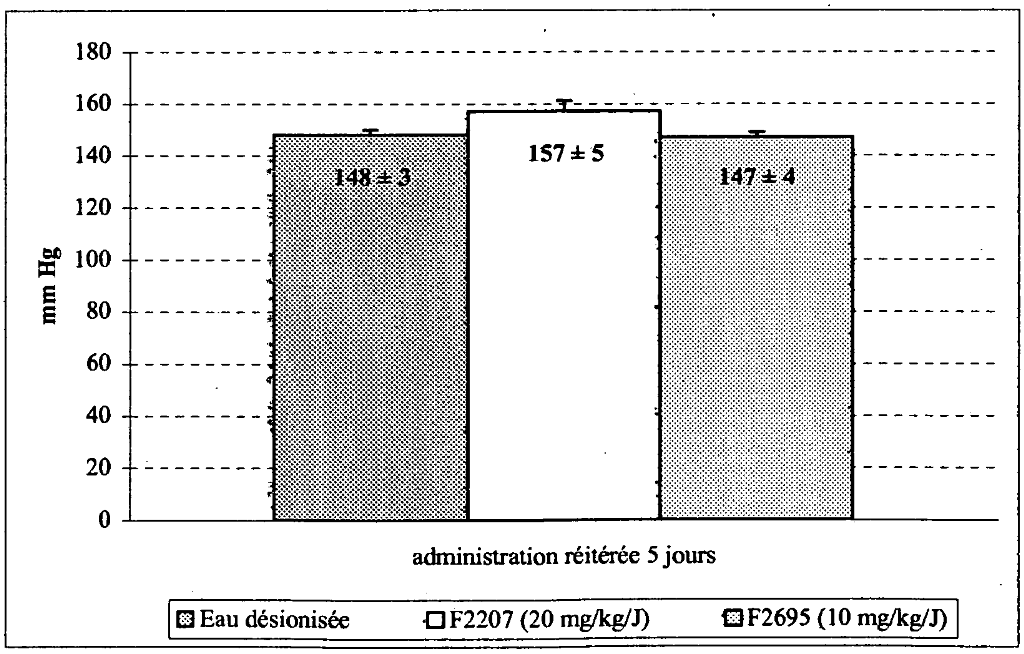


FIGURE 4

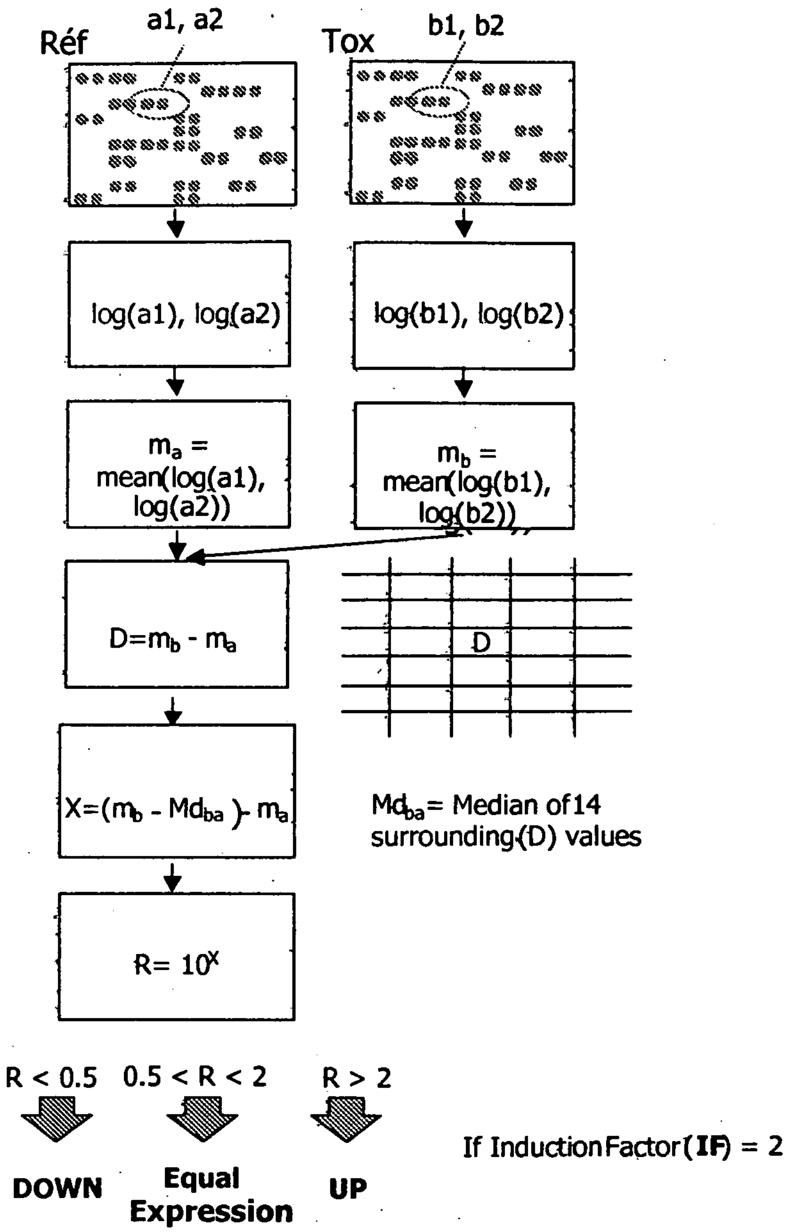
Réf
a1, a2
Tox
b1, b2
log(a1), log(a2)
log(b1), log(b2)
m_a = mean(log(a1), log(a2))
m_b = mean(log(b1), log(b2))
D=m_b - m_a
D
X=(m_b - Md_ba ) - m_a
Md_ba = Median of 14 surrounding (D) values
R= 10^X
R < 0.5
0.5 < R < 2
R > 2
DOWN
Equal Expression
UP
If Induction Factor (IF) = 2

FIGURE 5

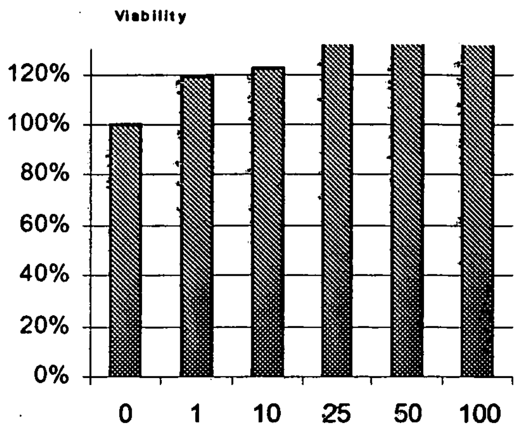


F2695

FIGURE 6A

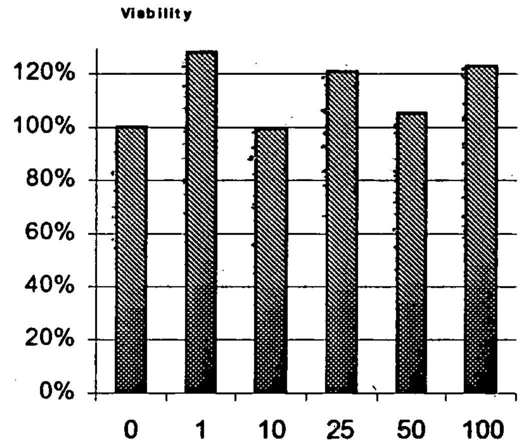


F2696

FIGURE 6B

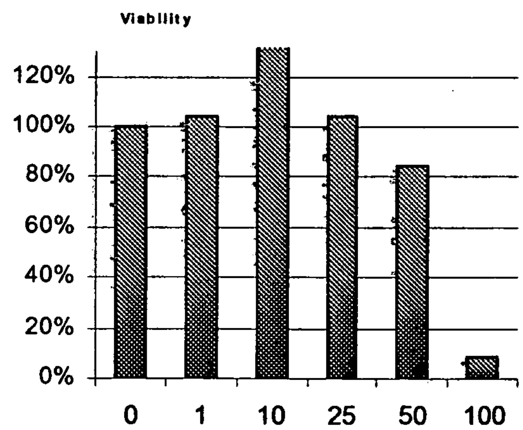


C218

FIGURE 6C

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4478836 A
- WO 0126623 A
- WO 0162236 A
- WO 9735574 A
- FR 2759290
- EP 939626 A


### Littérature non-brevet citée dans la description


- **Moret et al.** *Neuropharmacology,* 1985, vol. 24 (12), 1211-1219
- **Palmier et al.** *Eur J Clin Pharmacol,* 1989, vol. 37, 235-238
- **Bonnaud et al.** *Journal of Chromatography,* 1985, vol. 318, 398-403
- **Shuto et al.** *Tetrahedron letters,* 1996, vol. 37, 641-644
- **Grard et al.** *Electrophoresis,* 2000, vol. 21, 3028-3034
- **Doyle ; Hu.** *Advanced Synthesis and Catalysis,* 2001, vol. 343, 299-302
- **Nores et al.** *Thérapie,* 1987, vol. 42, 555-558
- **Meadoer-Woodruff et al.** *J. Clim. Psychopharmacol.,* 1988, vol. 8, 28-32
- The Diagnostic and Statistical Manual of Mental Disorders -IV (DSM-IV). American Psychiatric Association, 1995
- **Gray ; Whittaker.** *J. Anat.,* 1962, vol. 96, 79-97